(19) Europäisches Patentamt
European Patent Office
Office européen des brevets

(11) Veröffentlichungsnummer: **0 115 079**
**B1**

# (12) EUROPÄISCHE PATENTSCHRIFT

(45) Veröffentlichungstag der Patentschrift: **18.03.87**

(21) Anmeldenummer: **83113172.7**

(22) Anmeldetag: **28.12.83**

(51) Int. Cl.[4]: **C 07 D 295/08**, C 07 C 93/06, C 07 D 233/60, A 61 K 31/13, A 61 K 31/535, A 61 K 31/495, A 61 K 31/415

(54) **Substituierte 1-((3-(Amino)-propoxy)-phenyl)-1-(phenyl)-propan-1-ole, ihre Säureadditionssalze und quaternären Salze, Verfahren zu ihrer Herstellung und diese Verbindungen enthaltende Arzneimittel.**

(30) Priorität: **28.12.82 HU 419082**

(43) Veröffentlichungstag der Anmeldung: **08.08.84 Patentblatt 84/32**

(45) Bekanntmachung des Hinweises auf die Patenterteilung: **18.03.87 Patentblatt 87/12**

(84) Benannte Vertragsstaaten: **AT BE CH DE FR GB IT LI LU NL SE**

(56) Entgegenhaltungen:
**GB-A-1 478 185**
**US-A-3 075 014**

(73) Patentinhaber: **RICHTER GEDEON VEGYESZETI GYAR R.T., Gyömröi ut 19-21, H-1475 Budapest X (HU)**

(72) Erfinder: **Tóth, Edit, Dipl.- Ing., Szabolcska u. 7, H-1114 Budapest (HU)**
Erfinder: **Törley, József, Dipl.- Ing., Katona J. u. 41, H-1137 Budapest (HU)**
Erfinder: **Fekete, György, Dr., Széher u. 62, H-1021 Budapest (HU)**
Erfinder: **Szporny, László, Dr., Szabolcska u. 7, H-1114 Budapest (HU)**
Erfinder: **Vereczkey, László, Dr., Lajos u. 109, H-1036 Budapest (HU)**
Erfinder: **Pálosi, Eva, Dr., Vend u. 21, H-1025 Budapest (HU)**
Erfinder: **Klebovich, Imre, Dr., Karvaly u. 4, H-1125 Budapest (HU)**
Erfinder: **Vittay, Pál, Dr., József krt. 14, H-1085 Budapest (HU)**
Erfinder: **Görög, Sándor, Dr. Dipl.- Chem., Vajda P. u. 43, H-1089 Budapest (HU)**
Erfinder: **Hajdu, István, Dipl.- Chem., Tátra tér B/4, H-1205 Budapest (HU)**

(74) Vertreter: **Beszédes, Stephan G. Dr., Münchener Strasse 80a Postfach 1168, D-8060 Dachau (DE)**

EP 0 115 079 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue substituierte 1-{[3-(Amino)-propoxyl]-phenyl}-1-{phenyl} -propan-1-ole, ihre Säureadditionssalze und quaternären Salze, ein Verfahren zu ihrer Herstellung und diese Verbindung enthaltende Arzneimittel.

Gegenstand der Erfindung sind substituierte 1- {[ 3-(Ami-no)-propoxy]-phenyl}-1- {phenyl}-propan-1-ole der allgemeinen Formel

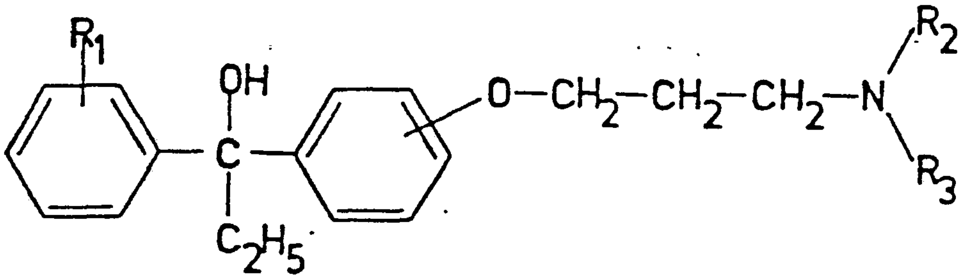

I ,

worin

$R_1$ für ein Halogenatom einen Trihalogenmethylrest oder einen, Alkoxy- oder Alkylrest mit je 1 bis 3 Kohlenstoffatom(en) steht,

$R_2$ einen Alkylrest mit 1 bis 3 Kohlenstoffatom(en) bedeutet und

$R_3$ einen Cycloalkylrest mit 3 bis 6 Kohlenstoffatonen darstellt oder

$R_2$ und $R_3$ zusammen mit den Stickstoffatom, an welches sie gebunden sind, einen, gegebenenfalls durch 1 oder mehr Alkylrest(e) mit 1 bis 4 Kohlenstoffatom(en) oder Benzylrest(e) substituierten und/oder gegebenenfalls als weiteres Heteroatom ein Sauerstoffatom oder ein weiteres Stickstoffatom aufweisenden, heterocyclischen Rest mit höchstens 8 Gliedern darstellt,

sowie ihre Säureadditionssalze und quaternären Salze.

Die Herstellung von Verbindungen ähnicher Struktur ist in folgenden Literaturstellen beschrieben: C.A. 22, 4101[1]; 35, 1781[2]; 40, 4712[5]; 42, P 1015 b; 47, 9548 e; 50, 12390 c; 50, 2509 i; 55, 17915 e; 55, 15413 b; 75, 103682 b; 76, P 119921 k; 82, 16477 q; 90, 86062 q; 92, 52927 b. Nirgends wird jedoch eine pharmakologische Wirkung der hergestellten Verbindungen erwähnt.

Ferner sind aus der britischen Patentschrift 1 478 185 gemäß deren Patentanspruch 1 substituierte Benzhydrolderivate, bei welchen der eine Benzolring unter anderen durch 1 oder mehr Halogenatom(e), Trihalogenmethylrest(e) und/oder geradkettige[n] und/oder verzweigte[n] gesättigter[n] uund/oder ungesättigte[n] aliphatische[n] Kohlenwasserstoffrest(e) mit 1 bis 6 Kohlenstoffatom(en) substituiert sein kann, sowie ferner aus ihren Beispielen 13 und 14 4-(β-Diäthylamino-äthoxy)-α-(äthyl)-benzhydrol und 4-(D-Diäthylaminoäthoxy)-α-(äthyl)-benzhydroläthobromid bekannt. Als pharmakologische Wirksamkeit dieser Verbindungen ist die Blockier- oder Induzierwirkung auf das Fremdsubstanzen umsetzende mikrosomale Enzymsystem angegeben. Für Substanzen, deren Inaktivierung durch die enzyminduzierende Wirkung der Verbindungen der britischen Patentschrift 1 478 185 beschleunigt wird, sind Bilirubin, Insektizide, Progesteron, Hexobarbital, Meprobamat und Bromsulfophthalein speziell erörtert.

Zwar können die Verbindungen der britischen Patentschrift 1 478 185 gemäß deren Patentanspruch 1 am einen Benzolring 1 oder mehr der Substituenten, welche $R_2$ in der Formel I der erfindungsgemäßen Verbindungen bedeuten kann, aufweisen, die erfindungsgemäßen Verbindungen sind jedoch im Gegensatz zu den Verbindungen der genannten Druckschrift, welche am zweiten Benzolring nicht substituiert sind, am zweiten Benzolring zwingend durch einen [ω -(Diäthylamino)-propoxy] -rest substituiert.

Zwar haben die Verbindungen der Beispiele 13 und 14 der britischen Patentschrift 1 478 185, welche nicht unter deren Patentanspruch 1 fallen, einen β-Diäthylaminoäthoxysubstituenten, welcher dem für den Fall, daß $R_2$ in der Formel I für einen Alkylrest steht und $R_3$ einen Cycloalkylrest bedeutet, in den erfindungsgemäßen Verbindungen vorliegenden { [N-(Alkyl)-N-(cycloalkyl)-amino ]-propoxy}-substituenten ähnlich ist, an ihrem einen Benzolring, auch von diesen bekannten Verbindungen, deren anderer Benzolring nicht substituiert ist, unterscheiden sich aber alle erfindungsgemäßen Verbindungen darin, daß auch ihr anderer Benzolring substituiert ist.

Weiterhin sind im der US-Patentschrift 3 075 014 substituierte Diphenylbutanole beziehungsweise -butenole und entsprechende höhere Alkanole beziehungsweise Alkenole beschrieben, wobei als niedrigste spezielle Alkanole Heptanole genannt sind. Als spezielle Indikationen ihrer pharmakologischen Wirkung sind die Behandlung von Hypercholesterinämie und Herzgefäßerkrankungen, welche durch hohe Cholesterinblutspiegel verschärft werden, sowie entzündungshemmende, östrogene und antiöstrogene Wirkungen angegeben.

Die erfindungsgemäßen Verbindungen unterscheiden sich von allen Verbindungen der US-Patentschrift 3 075 014 darin, daß die letzteren mindestens Butanole sind, während die erfindungsgemäßen Verbindungen zwingend Propanole sind.

Vorzugsweise ist das Halogen, für welches $R_1$ stehen kann, Chlor oder Flur.

Es ist auch bevorzugt, daß der Trihalogenmethylrest, für den $R_1$ stehen kann, ein Trifluormethylrest oder Trichlormethylrest ist.

Ferner ist es bevorzugt, daß der Alkoxyrest, für den $R_1$ stehen kann und/oder der beziehungsweise die Alkylrest(e), für den beziehungsweise die $R_1$ und/oder $R_2$ stehen kann beziehungsweise können, ein solcher beziehungsweise solche mit 1 oder 2, insbesondere 1, Kohlenstoffatom(en) ist beziehungsweise sind.

Weiterhin ist es bevorzugt, daß der Cycloalkylrest, für den $R_3$ stehen kann, ein solcher mit 5 oder 6, insbesondere 6, Kohlenstoffatomen ist.

Außerdem ist es bevorzugt, daß der heterocyclische Rest, den $R_2$ und $R_3$ zusammen mit dem Stickstoffatom, an welches sie gebunden sind, darstellen können, ein solcher mit 3 bis 8, insbesondere 5 oder 6, ganz besonders 6, Kohlenstoffatomen, vor allem ein Morpholinyl-, Piperazinyl-oder Imidazolylrest, ist.

Vorzugsweise ist beziehungsweise sind der beziehungswise die Alkylrest(e), durch welchen beziehungsweise welche der heterocyclische Rest, den $R_2$ und $R_3$ zusammen mit dem Stickstoffatom, an welches sie gebunden sind, darstellen können, substituiert sein kann, ein solcher beziehungsweise solche mit 1 bis 3, insbesondere 1 oder 2, ganz besonders 1, Kohlenstoffatom(en).

Bevorzugt ist der substituierte [3-(Amino)-propoxyl]-rest in der 4-Stellung des Benzolringes, an welchem er hängt.

Besonders bevorzugte erfindungsgemäße 1-{[ 3-(Amino)-propoxyl]-phenyl} -1-{phenyl} -propan-1-ole sind 1-(4-Chlor-phenyl)-1-{4-[3-(4-metyl-piperazin-1-yl)-propoxy]-phenyl}-propan-1-ol, 1-(3-trifluormethylphenyl)-1-{4-[3-(morpholin-1-yl)-propoxy]-phenyl}-propan-1-ol, 1-(4-Fluorphenyl)-1-{4-[3-(4-benzyl-piperazin-1-yl)-propoxy]-phenyl} -propan-1-ol, 1-(3-Chlor-phenyl)-1-{4-[3-(4-benzyl-piperazin-1-yl)-propoxy]-phenyl}-propaen-1-ol,1-(3-Trifluormethylphenyl)-1-{4-[3-(4-methyl-piperazin-1-yl)-propoxy]-phenyl} propan-1-ol, 1-(2-Methoxy-phenyl)-1-{4-[3-(4-methyl-piperazin-1-yl)-propoxy]-phenyl}-propan-1-ol, 1-(4-Chlorphenyl)-1-{4-[3-(4-benzyl-piperazin-1-yl)-propoxy]-phenyl}-propan-1-ol, 1-(3-Chlorphenyl)-1-{4[3-(4-methyl-piperazin-1-yl)-propoxy]-phenyl}-propan-1-ol, 1-(2-Methoxyphenyl)-1-{4-[3-(4-benzyl-piperazin-1-yl)-propoxy]-phenyl}-propan-1-ol, 1-(2-Methoxyphenyl)-1-{4-[3-(2-methyl-imidazol-1-yl)-propoxy]-phenyl}-propan-1-ol, 1-(2-Methoxyphenyl)-1-{4-[3-(imidazol-1-yl)-propoxy]-phenyl}-propan-1-ol und 1-(3-Triflourmethylphenyl)-1-{4-[-3-(N-methyl-N-cyclohexyl-amino)-propoxy]-phenyl}-propan-1-ol sowie ihre Salze.

Gegenstand der Erfindung ist auch ein Verfahren zur Herstellung der erfindungsgemäßen Verbindungen, welches dadurch gekennzeichnet ist, daß

a) am Benzolring 3-(amino)-propoxysubstituierte Propiophenone der allgemeinen Formel

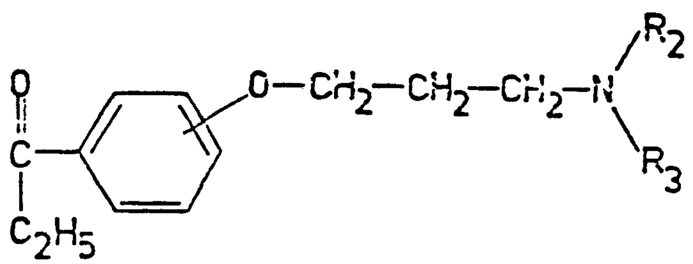

II ,

worin $R_2$ und $R_3$ wie oben festgelegt sind, mit eine substituierte Phenylgruppe aufweisenden metallorganischen Verbindungen der allgemeinen Formel

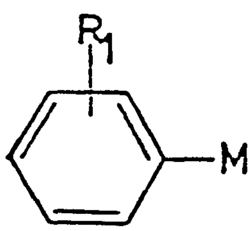

III ,

worin

$R_1$ wie oben festgelegt ist und

M für ein Alkalimetallatom, vorzugsweise Lithium-, Kalium- oder Natriumatom, oder einen Rest der Formel MgX mit X = Halogenatom steht,

umgesetzt werden oder

b) substituierte 1-{[3-(Halogen)-propoxyl-phenyl}-1-{phenyl}-propan-1-ole der allgemeinen Formel

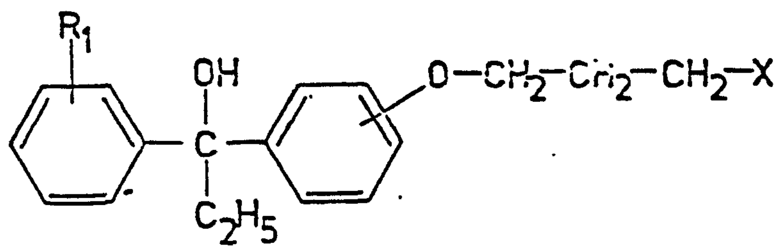

IV ,

worin
$R_1$ wie oben festgelegt ist und
X für ein Halogenatom steht,
mit sekundären Aminen der allgemeinen Formel

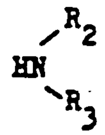

V ,

worin $R_2$ und $R_3$ wie oben festgelegt sind, vorzugsweise in Gegenwart von säurebindenden Mitteln, umgesetzt werden oder

c) substituierte [3-(Amino)-propoxy]-benzophenone der allgemeinen Formel

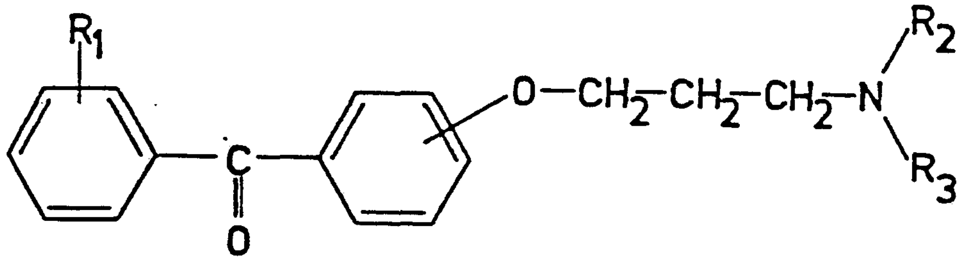

VI ,

worin $R_1$, $R_2$ und $R_3$ wie oben festgelegt sind, mit eine Äthylgruppe aufweisenden metallorganischen Verbindungen, vorzugsweise Äthylmagnesiumhalogeniden oder Äthyllithium, umgesetzt werden oder

d) am Benzolring substituierte Propiophenone der allgemeinen Formel

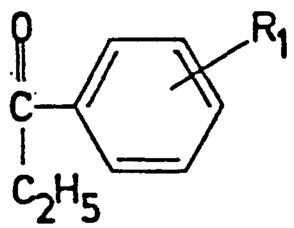

VII ,

worin $R_1$ wie oben festgelegt ist, mit eine {[3-(Amino) -propoxy] -phenyl}-gruppe aufweisenden Grignard-Verbindungen der allgemeinen Formel

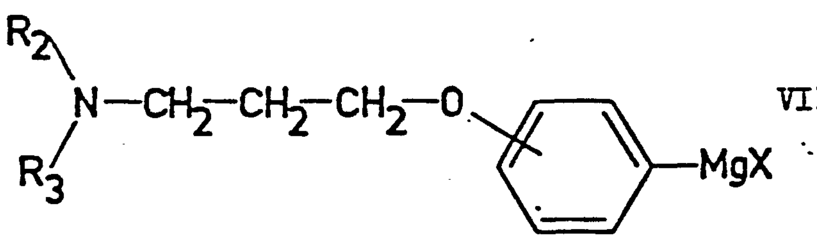

VIII ,

worin
$R_2$ und $R_3$ wie oben festgelegt sind und
X Halogen bedeutet,
ungesetzt werden oder

e) substituierte 1 -(Phenyl)-1 -(hydroxyphenyl) -propan-1-ole der allgemeinen Formel

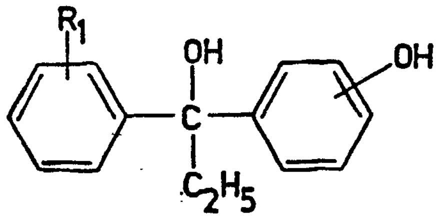

II ,

worin $R_1$ wie oben festgelegt ist, vorzugsweise in Form ihrer Alkaliphenolate oder quaternären Ammoniumphenolate, mit tertiären Aminen der allgemeinen Formel

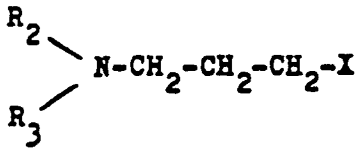

I ,

worin

$R_2$ und $R_3$ wie oben festgelegt sind und

X für einen Alkylsulfonyloxy- oder Arylsulfonyloxyrest oder ein Halogenatom steht

oder mit deren Salzen, vorzugsweise in Gegenwart von säurebindenden Mitteln, umgesetzt werden,

worauf gegebenenfalls in an sich bekannter Weise die erhaltenen 1-{[ 3-(Amino)-propoxyl-phenyl] -1-{phenyl} -propan-1-ole der allgemeinen Formel I mit Säuren in Säureadditionssalze oder mit Quaternisierungsmitteln in quaternäre Ammoniumsalze überführt werden oder gegebenenfalls die erhaltenen Säureadditionssalze der 1-{[3-(Amino)-pro-poxy]-phenyl} -1-{phenyl} -propan-1-ole der allgemeinen Formel I in die entsprechenden freien Basen der allgemeinen Formel I und/oder in andere Säureadditionssalze oder quaternäre Ammoniumsalze überführt werden.

Die Ausgangsverbindungen sind bekannt beziehungsweise können nach literaturbekannten Verfahren hergestellt werden. Die Ketone der allgemeinen Formeln (II), (VI) und (VII) können zum Beispiel mit der Friedel-Crafts-Ketonsynthese erhalten werden (G.A. Olah: Friedel-Crafts and related reactions, Band III/1, Ed.: Interscience Publishers, 1964, S. 1-63).

Die Verbindungen der allgemeinen Formel (III) und (VIII) erhält man zum Beispiel, indem man aus den entsprechend substituierten Arylhalogeniden in an sich bekannter Weise das Grignard-Reagens bildet (M.S. Kharash et al.: Grignard reactions of nonmetallic substances, W. Prentice-Hall. Inc., 1954, S. 5-90). Die ein Alkalimetall enthaltenden metallorganischen Verbindungen können zum Beispiel nach Houben-Weyl: Methoden der organischen Chemie, Bd. XIII/1, S- 134-159 sowie 389-405 (1970) hergestellt werden.

Die Verbindungen der allgemeinen Formel (IV) und (IX) sind zum Beispiel durch Umsetzen der entsprechend substituierten Propiophenone mit entsprechenden Grignard-Reagentien erhältlich (s. zum Beispiel das oben zitierte Buch von M.S. Kharash et al., S.. 138-143).

Gemäß einer bevorzugten Ausführungsform der Variante a) des erfindungsgemäßen Verfahrens setzt man die Propiophenone der allgemeinen Formel (II) in einem wasserfreien, inerten organischen Lösungsmittel mit der metallorganischen Verbindung der allgemeinen Formel (III), vorzugsweise dem entsprechend substituierten Phenylmagnesiumchlorid oder -bromid oder dem entsprechend substituierten Phenyllithium, um. Als Lösungsmittel verwendet man aprotische organische Lösungmittel, zum Beispiel die folgenden: aliphatische Äther, wie Diäthyläther, Di-n-butyläther, Diäthylenglykoldimethyläther, oder alicyclische Äther, wie Tetrahydrofuran oder Dioxan, aliphatische oder aromatische Kohlenwasserstoffe, zum Beispiel Ligroin, Benzol, Toluol, Xylol, ferner Dimethylsulfoxyd, Hexamethylphosphoramid oder Gemische der aufgeführten Lösungsmittel. Die metallorganische Verbindung wird in wenigstens äquimolarer Menge eingesetzt. Die Reaktion wird vorzugsweise unter Schutzgasatmosphäre, zum Beispiel unter Stickstoff- oder Argonatmosphäre, vorgenommen. Die Reaktionstemperatur liegt zwischen -60 °C und dem Siedepunkt des Lösungsmittels, vorzugsweise zwischen -30 und 100 °C. Nach Beendigung der Reaktion wird das metallorganische Zwischenprodukt zweckmäßig mit einer Ammoniumchloridlösung gespalten und die gebildete Verbindung der allgemeinen Formel (I) isoliert. Das Produkt kann in an sich bekannter Weise, zum Beispiel durch Destillieren oder Umkristallisieren, gereinigt werden.

Gemäß der Verfahrensvariante b) werden die Verbindungen der allgemeinen Formel (IV), in denen X vorzugsweise für ein Chlor-oder Bromatom steht, mit sekundären Aminen der allgemeinen Formel (V) umgesetzt. Die Umsetzung wird vorzugsweise in einem organischen Lösungsmittel sowie in Gegenwart eines Säureakzeptors vorgenommen.

Als Lösungsmittel kommen zum Beispiel in Betracht: Kohlenwasserstoffe, wie Ligroin, Benzol, Toluol, oder halogenierte Kohlenwasserstoffe, wie Chloroform, Äther, zum Beispiel Dioxan, Alkohole, wie Äthanol, Ketone, zum Beispiel Aceton oder Methylisobutylketon, Ester, wie Äthylacetat, Säureamide, zum Beispiel Dimethylformamid, oder Gemische der aufgeführten Lösungsmittel. Als Säureakzeptor verwendet man vorzugsweise anorganische oder tertiäre organische Basen, inbesondere Amine, oder aber einen Überschuß des Amins der allgemeinen Formel (V). Wird als Säureakzeptor eine tertiäre organische Base oder der Überschuß des Amins der allgemeinen Formel (V) verwendet, so können diese gleichzeitig als Lösungsmittel

wirken. Die Reaktionstemperatur liegt zwischen 20°C und dem Siedepunkt des Lösungsmittels. Nach Beendigung der Reaktion wird das Produkt abgetrennt. Zur Aufarbeitung gießt man das Reaktionsgemisch beispielsweise in Wasser und extrahiert mit einem organischen Lösungsmittel. Die organische Phase wird mit Wasser halogenfrei gewaschen, getrocknet und dann eingedampft. Das Rohprodukt kann durch Destillieren oder Umkristallisieren gereinigt werden.

Gemäß der Verfahrensvariante c) werden die Benzophenone der allgemeinen Formel (VI) mit wenigstens der äquimolaren Menge von vorzugsweise Äthylmagnesiumbromid oder -jodid oder von Äthyllithium umgesetzt. Die Umsetzung wird ähnlich wie die Umsetzung gemäß a) in einem inerten, wasserfreien organischen Lösungsmittel vorgenommen.

Gemäß der Verfahrensvariante d) werden Grignard-Verbindungen der allgemeinen Formel (VIII), insbesondere solche, in denen X für ein Bromatom steht, einem wasserfreien, inerten organischen Lösungsmittel mit wenigstens der äquimolaren Menge des Propiophenons der allgemeinen Formel (VII) ähnlich wie unter a) beschrieben umgesetzt.

Gemäß der Verfahrensvariante a) werden Verbindungen der allgemeinen Formel (IX), vorzugsweise in Form ihrer Alkaliphenolate oder quaternären Ammoniumphenolate, mit tertiären Aminen der allgemeinen Formel (X) kondensiert. Als tertiäre Amine kommen zum Beispiel Dialkylaminoalkylmesylate, -tosylate, -bromide und insbesondere -chloride in Frage, gegebenefalls in Form eines Hydrohalogenids. Die Reaktion wird vorzugsweise in Gegenwart eines Säureakzeptors in einem wasserfreien oder wasserhaltigen organischen Lösungsmittel vorgenommen. Als Lösungsmittel kommen in Frage: Ester, wie Äthylacetat; Äther, wie Dioxan, Tetrahydrofuran oder Diäthyläther; Kohlenwasserstoffe, zum Beispiel Ligroin, Benzol, Toluol, Xylol; halogenierte Kohlenwasserstoffe, wie Chloroform oder Chlorbenzol; Säureamide, wie Dimethylformamid; Ketone, zum Beispiel Aceton, Methyläthylketon, Methylisobutylketon; Alkohole, wie Äthanol oder Propanol. Die Phenolate der Verbindungen der allgemeinen Formel (IX) können in an sich bekannter Weise gebildet werden, zum Beispiel durch Umsetzen mit Alkalialkoholaten, -amiden, -hydriden, -hydroxyden, -carbonaten oder quaternären Ammoniumverbindungen. Als Säureakzeptor kommen vorzugsweise anorganische oder tertiäre organische Basen, zum Beispiel Natriumhydroxyd, Kaliumhydroxyd, Kaliumcarbonat, Triäthylamin und Pyridin, in Betracht. Die Umsetzung kann in Gegenwart eines Katalysators vorgenommen werden. Als Katalysator kann zum Beispiel ein Alkalihalogenid, vorzugsweise ein Alkalijodid, eingesetzt werden. Die Reaktionstemperatur kann innerhalb weiter Grenzen variieren, im allgemeinen arbeitet man bei Temperaturen zwischen 20 °C und dem Siedepunkt des Lösungsmittels.

Aus den Verbindungen der allgemeinen Formel (I) können gewünschtenfalls in an sich bekannter Weise Säureadditionssalze oder quaternäre Ammoniumsalze hergestellt werden. Zur Bildung der Säureadditionssalze sind anorganische und organische Säuren geeignet, zum Beispiel: Halogenwasserstoffsäuren, wie Salzsäure oder Bromwasserstoffsäure, Schwefelsäure, Phosphorsäure, Ameisensäure, Essigsäure, Propionsäure, Oxalsäure, Glykolsäsure, Maleinsäure, Fumarsäure, Bersteinsäure, Weinsäure, Ascorbinsäure, Citronensäure, Apfelsäure, Salicylsäure, Milchsäure, Benzoesäure, Zimtsäure, Asparaginsäure, Glutaminsäure, N-Acetylasparaginsäure, N-Acetylglutaminsäure, Alkylsulfonsäuren, zum Beispiel Methansulfonsäure, Arylsulfonsäuren, wie p-Toluolsulfonsäure.

Zur Salzbildung löst man zum Beispiel die Verbindung der allgemeinen Formel (I) in einem inerten Lösungsmittel, zum Beispiel in Äthanol, und gibt zu der Lösung die entsprechende Säure. Das gebildete Salz fällt man zweckmäßig mit einem mit Wasser nicht mischbaren Lösungsmittel, zum Beispiel mit Diäthyläther, aus. Zur Bildung der quaternären Salze verwendet men zweckmäßig niedere Alkyl-, Alkenyl- oder Benzylhalogenide oder Alkylsulfate. Die Selzbildung erfolgt in einem organischen Lösungsmittel, zweckmäßig in Aceton, Acetonitril, Äthanol oder in Gemischen der genannten Lösungsmittel, bei Temperaturen zwischen Raumtemperatur und der Siedetemperatur des Lösungsmittels. Das quaternäre Salz kann zum Beispiel durch Filtrieren abgetrennt und erforderlichenfalls durch Umkristallisieren gereinigt werden.

Die Verbindungen der allgemeinen Formel (I) sind zur Behandlung akuter Äthanolintoxikation geeignet und für diesen Zweck ausgedehnt verwendbar. Die akute Alkoholintoxikation ist vor allem durch Euphorie, allgemeine Stimuliertheit, Ataxie, Somnolenz und paralytische Zustände charakterisiert. Die Gefahren dieses toxischen, krankhaften Zustandes sind bekannt und nicht zu vernachlässigen. Die intoxierte Person gefährdet ihre Umgebung (zum Beispiel Trunkenheit am Steuer) und sich selbst.

Die akute Alkoholintoxikation ist für den isohämischen Gehirninfarkt ein bedeutender Risikofaktor (Hillbom, M. u.a.: Lancet 2, 1181 /1978/; Stroke 12, 422/1981/). Für den alkoholintoxierten Zustand gibt es kein Antidotum. Die durch Alkohol ausgelöste lokomotorische Hyperaktivität wird an Mäusen durch α-Methyl-para-thyrosin in einem Dosisbereich normalisiert, in dem die Verbindung die spontane lokomotorische Aktivität der Tiere beeinträchtigt (Carlsson, A. u. a.: Psychopharm. 26, 307/1972/). Die narkotisierende Wirkung des Alkohols wird durch Stimulantien (Coffein, Amphetamin) verringert, die motorische Inkoordination (Ataxie) jedoch gleichzeitig prolongiert (Wallagsen, H. u.a: Actions of alcohol, Amsterdam, Elsevier, 1970; Rech, R.H. u.a: Ann.N.Y. Acad. Sci. 28, 426/1976/; Todzy, I. u.a: Psychopharm. 59, 143/1978/). Die alkoholische Intoxikation, die Narkose wird durch L-Cystein verkürzt (Sprince, H. u. a: Agents and Actions, 4, 125/1974/; Nagasawa, H.T. u.a: Life Sci. 17, 707/1975/); L-Cystein wurde zu den folgenden Untersuchungen über Schlaf unter Alkoholeinfluß als Referenzsubstanz verwendet.

Da Alkohol keine Substanz ist, welche durch Induzierung der Wirkung eines Enzymes schneller inaktiviert werden kann, hat die pharmakologische Wirksamkeit der Verbindungen der britischen Patentschrift 1 478 185 mit den pharmakologischen Wirkungen gegen akute Alkoholintoxikation der erfindungsgemäßen

Verbindungen nichts zu tun.

Zur Messung der Änderung der Dauer der durch Alkohol hervorgerufenen Narkose wurden 16 Stunden lang ausgehungerte, 160-180 g schwere Hann.- Wistar-Ratten beiderlei Geschlechts verwendet. Jede Gruppe bestend aus 10 Tieren, die mit entsprechenden Dosen der Testverbindungen oral behandelt wurden. Eine Stunde nach der Behandlung wurde den Tieren in einer Dosis von 3,5 g/kg intraperitoneal Äthanol appliziert. Die Schlafdauer der Tiere wurde vom Ausfall des Aufrichtreflexes (Righting-Reflexes) bis zur spontanen Korrektur der Körperhaltung gemessen. Für jede Gruppe wurde die durchschnittliche Schlafzeit, die Stendardabweichung und das Ergebnis in Prozent der Kontrolle berechnet. Die Kontrolle erhielt Placebo und ebenfalls 3,5 g/kg Alkohol. Die Ergebnisse sind in der folgenden Tabelle angegeben, in der $\bar{x}$ $\pm$S.E Durchschnittswert $\pm$ Standardabweichung bedeutet. Die Schlafdauer der Kontrolle betrug 82,6 $\pm$ -8,21 min ($\bar{x}$ $\pm$ S.E.).

A = 1-(2-Methoxyphenyl)-1-{4-[3-(4-methylpiperazin-1-yl)-propoxy]-phenyl-propan-1-ol

B = 1-(3-Chlorphenyl)-1-{4- [3-(4-benzyl-piperazin-1-yl)-propoxy]-pheny}-propan-1-ol-dihydrocitrat

C = 1-(4-Chlorphenyl)-1-{4-[3-(4-methyl-piperazin-1-yl)-propoxy]-phenyl}-propan-1-ol

Tabelle

| Behandlung | Dosis mg/kg | Narkosedauer in Prozent der Kontrolle (Kontrolle $\pm$S.E.%) |
|---|---|---|
| A | 40 | 65 $\pm$ 8,7 |
| B | 5 | 53 $\pm$ 3,7 |
| | 10 | 32 $\pm$ 10,1 |
| C | 40 | 51 $\pm$ 8,2 |
| L-Cystein | 500 | 63 $\pm$ 4,7 |
| | 1000 | 66 $\pm$ 5,9 |
| Kontrolle | | 100 $\pm$ 9,9 |

Aus den Daten der Tabelle ist ersichtlich, daß die Verbindungen der allgemeinen Formel (1) die Dauer des durch Äthanol ausgelösten Narkoseschlafes beträchtlich verkürzen. Während die Erhöhung der L-Cysteindosis auf 1 g/kg keine stärkere Verringerung der Narkosedauer hevorruft, wirken die erfindungsgemäßen Verbindungen dosisabhängig und erreichen beziehungsweise übertreffen die Wirkung des L-Cystein in einer zehn- bis nundertmal geringeren Dosis.

Die akute Toxizität der Verbindungen wurde an 160-180 g schweren Wistar-Ratten beiderlei Geschlechts untersucht. Die Tiere wurden mit 500 mg/kg der jeweiligen Testverbindung als einmalige Dosis oral behandelt und dann 14 Tage lang beobachtet. Kein einziges verendete.

Durch Untersuchungen auf die im folgenden angegebene Weise wurde festgestellt, daß die Verbindungen der allgemeinen Formel (I) auch in einer Dosis von 160 mg/kg keinerlei sonstige Wirkungen auf das Zentralnervensystem ausüben: Elektroschock (Swinyard, E.A., Brown, W.C., Goodman, L. S.: J. Pharmacol. Exp. Ther. 106, 319/1952/), Metrazolkrampf (Everett, G.M., Richards, R,K.: J. Pharmacol. Exp. Ther. 81, 402/1944/), Thiosemicarbazidkrampf (Da Vanzo, J.P., Greig, M.E., Cormin, M.A. Amer. J. Physiol. 201, 833/1961/), Strychninkrampf (Kerley, T.L., Richards, A.G., Begley, R.W. Abreu, B.B., Wesver, L.C: J. Pharmacol. Exp. Ther. 132, 360/1961/), Nikotinkrampf (Stone, C.A., Mecklenburg, K.L., Torhans, M.L: Arch. Int. Pharmacodyn. 117, 419/1958/), Drehstab (Kinnard, W.J., Carr, C.J: J. Pharmacol. Exp. Ther. 121, 354/1957/), Physostigminletalität (Nose, T. und Kojims, M.: Europ. J. Pharmacol. 10, 83/1970/), yohimbinpotenzierende Wirkung (Quinton, R.M: Brit. J. Pharmacol. 21, 51 /1963/), shmerzstillende Wirkung (Bianchi, C., Franceschini, J.: Brit. J. Pham. Chemother. 9, 280/1954/).

Die erfindungsgemäßen Verbindungen kompensieren die Wirkung des Alkohols auf das Zentralnervensystem in selektiver Weise und verkürzen die Narkosedauer um 50-70 %. Die Toxizität der Verbindungen ist gering, ihr therapeutischer Index ist gut.

Die erfindungsgemäßen Verbindungen können zu Arzneimittelpräparaten zubereitet werden. Die Präparate können oral, rektal und/oder parenteral verabreicht werden. Zur oralen Darreichung können Tabletten, Dragees oder Kapseln hergestellt werden. Bei der Herstellung von oral verabreichbaren Formen werden als Streckmittel zur Beispiel Milchzucker oder Stärke verwendet. Als Binde- und Granuliermittel kommen zum Beispiel Gelatine, Carboxymethylcellulose-natrium Methylcellulose, Polyvinylpyrrolidon oder Stärkekleister in Betracht. Als Sprengmittel werden in erster Linie Kartoffelstärke oder mikrokristalline Cellulose zugesetzt, jedoch können beispielsweise auch Ultraamylopektin oder Formaldehydcasein verwendet werden. Als Antihaftmittel und Gleitmittel kommen z.B. Talk, kolloidale Kieselsäure, Stearin, Ca-und Mg-Stearat in Frage.

Die Tabletten können zum Beispiel durch Naßgranulieren und anschließendes Pressen hergestellt werden. Das Gemisch aus Wirkstoff und Füllstoffen sowie degebenfalls ein Teil des Sprengmittels werden mit der wäßrigen, alkoholischen oder wäßrig-alkoholischen Lösung der Bindemittel in einer geeigneten Vorrichtung granuliert, und das Granulat wird getrocknet. Zu dem trockenen Granulat werden der Rest des Sprengmittels, ferner die Antihaftmittel und das Gleitmittel gegeben, und das Gemisch wird zu Tabletten gepreßt.

Gegebenenfalls können die Tabletten zur Erleichterung der Dosierung mit Teilungsmarkierungen versehen werden. Die Tabletten können auch unmittelbar durch Pressen eines Gemisches aus dem Wirkstoff und geeigneten Hilfsstoffen hergestellt werden.

Die Tabletten können gewünschtenfalls unter Verwendung der in der Arzneimittelherstellung üblichen Schutz-, Geschmacks- und Farbstoffe, zum Beispiel Zucker, Cellulosederivate, wie Methyl- oder Äthylcellulose oder Carboxymethylcellulosenatrium Polyvinylpyrrolidon, Calciumphosphat, Calciumcarbonat, Lebensmittelfarbstoffe, Lebensmittelfarblacke, Aromastoffe und Eisenoxydpigmente, auf die übliche Weise dragiert werden.

Zur Herstellung von Kapsel wird das Gemisch aus Wirkstoff und Füllstoffen in Kapseln gefüllt.

Zur rektalen Anwendung werden Suppositorien hergestellt. Diese enthalten außer dem Wirkstoff eine Trägermasse, das sog. Adeps pro suppositori. Als dieses kommen Pflanzenfette, zum Beispiel gehärtete Pflanzenöle, die Triglyceride von Fettsäuren mit 12-18 Kohlenstoffatomen, vorzugsweise die Trägerstoffe der Merkenbezeichnung Witepsol®, in Betracht. Der Wirkstoff wird in der geschmolzenen Trägermasse homogen verteilt und das erhaltene Gemisch zu Suppositorien vergossen.

Zum parenteralen Verabreichung werden Präparate in Injektionslösungsform hergestellt. Dazu werden die Wirkstoffe gegebenenfalls in Gegenwart von Lösungsvermittlern, wie Polyoxyäthylensorbitanmonolaurat, -monooleat oder -monostearat (Tween 20® Tween 60®, Tween 80®, in destilliertem Wasser und/oder unterschiedlichen organischen Lösungsmitteln, zum Beispiel Glykoläthern, gelöst. Die Injektionslösungen können ferner unterschiedliche Hilfsstoffe, zum Beispiel Konservierungsstoffe, wie Benzylalkohol p-Oxybenzoesäuremethyl- oder -propylester, Benzalkoniumchlorid oder Phenylmercuriborat, zum Binden von Metallspuren Komplexbildner, Äthylendiamin-tetraessigsäre, Puffersubstanzen oder Stoffe zum Einstellen des pH-Wertes sowie gegebenenfalls lokalanästhetisch wirksame Substanzen, wie Lidocain, enthalten. Die Injektionslösungen werden vor dem Abfüllen filtriert und, nachdem sie in Ampullen gefüllt wurden, sterilisiert.

Die Tagesdosis beträgt, abhängend vom Zustand des Kranken, 0,1-300 mg/kg, vorzugsweise 2,0-160 mg/kg, und wird zweckmäßig in kleineren Einzeldosen verabreicht.

Die Erfindung wird im folgenden an Hand von Ausführungsbeispielen näher erläutert.

**Beispiel 1**

1-(4-Chlorphenyl)-1-{4-[3-(4-methyl-piperazin-1-yl)-propoxy]-phenyl}-propan-1-ol

Zu dem aus 2,2 g Magnesiumspänen und 28,2 g 4- [3-(4-Methyl-piperazin-1-yl)-propoxy]-brombenzol in 170 ml wasserfreiem Tetrahydrofuran bereiteten Grignard-Reagens wird bei 20°C tropfenweise die Lösung von 10 g p-Chlorpropiophanon in 30 ml Tetrahydrofuran gegeben, und das Reaktionsgemisch 2 Stunden lang gekocht. Nach dem Abkühlen wird mit 20 %iger wäßriger Ammoniumchloridlösung hydrolytisch zersetzt. Das Tetrahydrofuran wird unter vermindertem Druck abdestilliert. Der Rückstand wird mit Benzol extrahiert, die organische Phase mit Wasser neutral gewaschen, über wasserfreiem Magnesiumsulfat getrocknet und das Lösungsmittel im Vakuum abdestilliert. Nach Kristallisieren des Rückstandes aus n-Hexan erhält man 17,1 g der Titelverbindung, die bei 124-125 °C schmilzt.

Elementaranalyse für die Summenformel $C_{23}H_{31}C1H_2O_2$

berechnet, %: C 68,55 H 7,75 Cl 8,90 N 6,95

gefunden, %: C 68,63 H 7,63 Cl 9,11 H 6,88.

Zu der mit wasserfreiem Äthanol bereiteten Lösung der Base wird die berechnete Menge salzsauren Äthanols gegeben und das Gemisch mit wasserfreiem Äther verdünnt. Das ausgefallene Dihydrochlorid der Base wird abfiltriert und getrocknet. Schmelzpunkt: 238-239°C.

Zu der mit wasserfreiem Äthanol bereiteten Lösung der freien Base wird die äthanolische Lösung der Äquivalenten Menge Citronensäure gegeben. Bei Zusatz von wasserfreiem Äther fällt das Dinydrocitrat kristallin aus. Es wird abfiltriert und getrocknet. Schmelzpunkt; 87-89 °C.

**Beispiel 2**

1-(3-Trifluormethylphenyl)-1-{4-[3-(morpholin-1-yl)-propoxy]-phenyl}-propan-1-ol

Ein Gemisch aus 18,6 g 1-(3-Trifluormethylphenyl)-1-[4-(3-chlorpropoxy)-phenyl]-propan-1-ol und 26,2 ml wasserfreiem Morpholin wird unter Rühren am Rückfluß 2 Stunden lang gekocht. Nach dem Abkühlen wird das Morpholin unter vermindertem Druck aus dem Reaktionsgemisch abdestilliert. Zu dem Rückstand gibt man Wasser und Benzol. Nach Trennung der Phasen wird die organische Phase mit Wasser gewaschen, über wasserfreiem Kaliumcarbonat getrocknet und das Benzol im Vakuum abdestilliert. Nach Kristallisieren des Rückstandes aus n-Hexan erhält man 18,2 g der Titelverbindung, die bei 68-70°C schmilzt.

Elementaranalyse für die Summenformel $C_{23}H_{28}P_3NO_3$

berechnet, %: C 65,23 H 6,66 P 13,46 N 3,31

gefunden, %: C 65,26 H 6,78 F 13,54 N 3,50.

**Beispiel 3**

1-(4-Fluorphenyl)-1-{4-[3-(4-benzyl-piperazin-1-yl)-propoxy]-phenyl}-propan-1-ol

Zu dem aus 2,4 g Magnesiumspänen und 11 g Äthylbromid in 40 ml wasserfreiem Äther hergestellten Grignard-Reagens wird bei -30° C tropfenweise die Lösung von 10,8 g 4-Fluor-4'-[3-(4-benzyl-piperazin-1-yl)-propoxy]-benzophenon in 200 ml wasserfreiem Äther gegeben. Das Reaktionsgemisch wird bei 0° C 30 Minuten lang gerührt und dann eine Stunde lang am Rückfluß gekocht. Nach dem Abkühlen wird das Gemisch in eiskalte wäßrige Annomiumchloridlösung gegossen. Die organische Phase wird abgetrennt und die wäßrige Phase mit Äther extrahiert. Die vereinigten ätherischen Phasen werden mit Wasser neutral gewaschen, über wasserfreiem Magnesiumsulfat getrocknet und dann im Vakuum eingedampft. Durch Kristallisieren des Rückstandes aus Isopropyläther erhält man 5,3 g der Titelverbindung, die bei 119-121 °C schmilzt. Elementaranalyse fü die Summenformel $C_{29}H_{35}FN_2O_2$

berechnet, %: C 75,29 H 7,63 F 4,11 N 6,06

gefunden, %: C 75,35 H 7,67 F 4,30 N 6,17.

**Beispiel 4**

1-(3-Chlorphenyl)-1-{4-[-(4-benzyl-piperazin-1-yl)-propoxy]-phenyl}-propan-1-ol

Zu dem aus 2,5 g Magnesisumspänen und 19,1 g 3-phlorbrom-benzol in 80 ml Tetrahydrofuran bereiteten Grignard-Reagens gibt man under schwachem Sieden am Rückfluß tropfenweise die Lösung von 25,6 g 4-[3-(4-Benzyl-piperazin-1-yl)-propoxyl-propiophenon in 55 ml wasserfreiem Tetrahydrofuran. Das Reaktionsgemisch wird weitere 2 Stunden lang eben am Sieden gehalten und dann nach dem Abkühlen in eiskalte wäßrige Ammoniumchloridlösung gegossen. Die Phasen werden voneinander getrennt, und die wäßrige Phase wird mit Tetrahydrofuran extrahiert. Die vereinigten organischen Phasen werden mit gesättigter wäßriger Kochsalzlösung neutral gewaschen und über wasserfreiem Kaliumcarbonat getrocknet. Das Lösungsmittel wird unter vermindertem Druck abdestilliert. Der Rückstand wird im Vakuum fraktioniert. Man erhält 20,1 g der Titelverbindung. Siedepunkt: 252-254 °C/6,6 Pa.

Elementranalyse für die Summenformel $C_{29}H_{35}C1N_2O_2$

berechnet, % C 72,70 H 7,36 Cl 7,40 N 5,85

gefunden, % C 72,74 H 7,55 Cl 7,28 N 5,91.

Das Dihydrocitrat schmilzt bei 67-69 °C.

**Beispiel 5**

1-(3-Trifluormethylphenyl)-1-{4-[3-(4-methyl-piperazin-1-yl)-propoxy]-phenyl}-propan-1-ol

Zu 200 ml einer 0,5 molaren, ätherischen Lösung von 3-Trifluormethylphenyl-lithium wird unter Argonatmosphäre bei -30°C tropfenweise die Lösung von 8,7 g 4- [3-(4-Methyl-piperazin-1-yl)-propoxy]-propiophenon in 270 ml wasserfreiem Äther gegeben. Das Reaktionsgemisch wird 3 Stunden lang gerührt und dann mit 20 %iger wäßriger Ammoniumchloridlösung versetzt. Die wäßrige Phase wird mit Äther extrahiert. Die vereinigten ätherischen Phasen werden über waßserfreiem Magnesiumsulfat getrocknet, und der Äther wird im Vakuum abdestilliert. Der Rückstand wird aus einem Gemisch von n-Hexan und Benzol kristallisiert. Man erhält 3,6 g der Titelverbindung, die bei 129-130 °C schmilzt.

Elementaranalyse für die Summenformel $C_{24}H_{31}F_3N_2O_2$

berechnet, %: C 66,03 H 7,16 P 13,06 H 6,42

gefunden, %: C 66,15 H 7,32 F 13,00 N 6,34.

**Beispiel 6**

1-(2-Methoxyphenyl)-1-{4-[3-(4-methyl-piperazin-1-yl)-propoxy]-phenyl}-propan-1-ol

Ein Gemisch aus 12,9 g 1-(2-Methoxyphenyl)-1-(4-hydroxy-phenyl)-propan-1-ol, 14 g wasserfreiem Kaliumcarbonat, 9,7 g 3-(4-Methyl-piperazin-1-yl)-propylchorid, 0,85 g Tetrabutylammoniumhydrogensulfat und 140 ml Äthylacetat wird am Rückfluß unter Rühren 20 Stunden lang gekocht. Nach dem Abkühlen wird das Lösungsmittel unter vermindertem Druck abdestilliert. Der Rückstand wird mit Wasser versetzt und mit Benzol extrahiert. Die organische Phase wird mit Wasser gewaschen, über wassenfreiem Magnesiumsulfat getrocknet und das Lösungsmittel unter vermindertem Druck abdestilliert. Durch Kristallisieren des Rückstandes aus n-Hexan erhält man 14,5 g der Titelverbindung, die bei 112-113 ° C schmilzt. Elementaranalyse für die Summenformel $C_{24}H_{34}N_2O_2$

berechnet, %: C 72,32 H 8,60 N 7,03

gefunden %: C 72,27 H 8,73 N 7,15. Das Dihydrocitrat schmilzt bei 105-107°C

**Beispiel 7**

1-(4-Chlorphenyl)-1-{4-[3-(4-benzyl-piperazin-1-yl)-propoxy ]-phenyl}-propan-1-ol

Ein Gemisch aus 6,6 g 1-(4-Chlorphenyl)-1-(4-hydroxy-phenyl)-propan-1-ol, 11,5 g wasserfreiem Kaliumcarbonat, 0,2 ml 40 %iger Tetrabutylammoniumhydroxydlösung, 6,3 g 3-(4-Benzyl-piperazin-1-yl)-propylchlorid und 70 ml Methyläthylketon wird 4 Stunden lang gekocht. Das Lösungsmittel wird unter vermindertem Druck abdestilliert und der Rückstand mit Benzol und Wasser versetzt. Die organische Phase wird mit Wasser neutral gewaschen, über wasserfreiem Kaliumcarbonat getrocknet und das Benzol im Vakuum abdestilliert. Der Rückstand wird in wasserfreiem Äthanol gelöst und zu der Lösung die äthanolische Lösung von 2,85 g Fumarsäure gegeben. Das ausgefallene Hydrofumarat wird abfiltriert und aus einem Gemisch von Methanol und Dimethylformamid kristallisiert. Man erhält 11,2 g Produkt, das bei 205-207 °C schmilzt. Aus dem Monohydrofumarat wird die Base mit Ammoniumhydroxyd freigesetzt.

Elementaranalyse für die Summenformel $C_{29}H_{35}ClN_2O_2$
berechnet, %: C 72,70 H 7,36 Cl 7,40 N 5,85
gefunden, %: C 72,66 H 7,33 Cl 7,58 N 5,91.

**Beispiel 8**

1-(4-Chlorphenyl)-1-{4-[3-(4-methyl-piperazin-1-yl)-propoxy]-phenyl}-propan-1-ol-dimethojodid

Ein Gemisch aus 4 g der Base, 20 ml Aceton und 5,6 g Methyljodid wird am Rückfluß eine Stunde lang schwach am Sieden erhalten und dann unter Kühlen stehen gelassen. Die ausgefallenen Kristalle werden abfiltriert und aus 95 %igem Äthanol umkristallisiert. Man erhält 5,1 g der quaternären Verbindung, die bei 186-188 °C schmilzt. In analoger Weise wird das 1-(2-Methoxyphenyl)-1-{4- [3-(4-methyl-piperazin-1-yl)-propoxy ]-phenyl}-propan-1-ol-dimethojodid hergestellt, das unter Zersetzung bei 228-230 °C schmilzt. Durch entsprechende Wahl der Ausgangsverbindungen werden auf die in den Beispielen 1-7 beschriebene Weise die folgenden Verbindungen hergestellt.

1-(3-Chlorphenyl)-1-{4-[3-(4-methyl-piperazin-1-yl)-propoxy ]-phenyl}-propan-1-ol, Schmelzpunkt: 110-111°C
Elementaranalyse. für die Summenformel $C_{23}H_{21}ClN_2O_2$
berechnet, %: C 68,55 H 7,75 Cl 8,80 N 6,95
gefunden, %: C 68,66 H 7,82 Cl 8,88 N 7,13

1- (2-Methoxyphenyl)-1-{4- [3-(4-benzyl-piperazin-1-yl)-propoxy]-pheny}-propan-1-ol
Schmelzpunkt: 63-64 °C
Elementaranalyse für die Summenformel $C_{30}H_{38}N_2O_3$
berechnet, %: C 75,91 H 8,07 N 5,90
gefunden, %: C 75,84 H 8,14 N 6,11

1-(2-Methoxyphenyl)-1-{4-[3-(2-methyl-imidazol-1-yl)-propoxy ]-phenyl}-propan-1-ol
Schmelzpunkt: 90-91 °C
Elementaranalyse für die Summenformel $C_{23}H_{28}N_2O_3$
berechnet, %: C 72,60 H 7,42 N 7,36
gefunden, %: C 72,55 H 7,61 N 7,55

1-(2-Methoxyphenyl)-1-{4-[3-(imidazol-1-yl)-propoxy]-phenyl}-propan-1-ol
Schmelzpunkt: 67-68 °C
Elementaranalyse für die Summenformel
$C_{22}H_{26}N_2O_3$
berechnet, %: C 72,10 H 7,15 N 7,64
gefunden, %: C 72,17 H 7,28 N 7,71

1-(Trifluormethylphenyl)-1-{4-[3-(N-methyl-N-cyclo-hexylamino)-propoxy]-phenyl} -propan-1-ol
Schmelzpunkt: 75-76 °C
Elementaranalyse für die Summenformel $C_{26}H_{34}P_3NO_2$
berechnet, %: C 69,46 H 7,62 F 12,68 N 3,12
gefunden, %: C 69,60 H 7,69 F 12,85 N 3,23.

**Beispiel 9**

Aus den erfindungsgemäßen Verbindungen können zum Beispiel die folgenden Arzneimittelpräparate hergestellt werden.

<u>Tabletten</u>

Zusammensetzung einer Tablette:
Wirkstoff 100,0 mg
Lactose 184,0 mg
Kartoffelstärke 80,0 mg

Polyvinylpyrrolidon 8,0 mg
Talk 12,0 mg
Magnesiumstearat 2,0 mg
Aerosil® (kolloidales $SiO_2$) 2,0 mg
Ultraamylopektin 12,0 mg

Aus den aufgeführten Stoffen werden durch Naßgranulieren und Pressen Tabletten eines Gewichtes von 400 mg hergestellt.

Wirkstoff: 1-(3-Chlorphenyl)-1-{4-[3-(4-benzyl-piperazin-1-yl)-propoxy ]-phenyl}-propan-1-ol-dihydrocitrat.

Dragees

Die auf die beschriebene Weise hergestellten Tabletten werden in an sich bekannter Weise mit einer aus Zucker und Talk bestehenden Schicht überzogen und dann mit einem Gemisch aus Bienenwachs und Carnaubawachs poliert. Gewicht eines Dragees: 500 mg.

Kapseln

Zusammensetzung der Füllung einer Kapsel:
Wirkstoff 50,0 mg
Lactose 100,0 mg
Talk 2,0 mg
Kartoffelstärke 30,0 mg
mikrokristalline Cellulose 8,0 mg

Der Wirkstoff wird gründlich mit den Hilfsstoffen vermischt, das Gemisch durch ein Sieb der Maschenweite 0,32 mm gesiebt und dann in Hartgelatinekapsel der Größe 4 gefüllt. Wirkstoff: 1-(4-Chlorphenyl)-1-{4-[3-(4-methyl-piperazin-1-yl)-propxy]-pheny} propan-l-ol.

Suppositorien

Zusammensetzung eines Suppositoriums:
Wirkstoff 100,0 mg
Lactose 200,0 mg
Grundmasse (z.B. Witepsol® H) 1700,0 mg

Die Grundmasse wird geschmolzen und die Schmelze auf 35 °C gekühlt. Der Wirkstoff wird gründlich mit der Lactose vermischt und das Gemisch in einem Homogenisator mit der Schmelze homogenisiert. Das erhaltene Gemisch wird in gekühlte Suppositorienformen gegossen. Gewicht eines Suppositoriums: 2000 mg.

Wirkstoff: 1-(3-Chlorphenyl)-1-{4-[3-(4-benzyl-piperazin-1-y 1)-propoxy ]-phenyl}-propan-1-ol.

Suspension

100 ml Suspension enthalten:
Wirkstoff 1,0 g
Natriumhydroxyd 0,26 g
Citronensäure 0,30 g
Nipagin® (Natriumsalz des
4-Hydroxybenzoesäuremethylesters) 0,10 g
Carbopol® 940 (Polyacrylsäure) 0,30 g
Äthanol (96 %ig) 1,00 g
Himbeeraroma 0,60 g
Sorbit (70 %ige wäßrige Lösung) 71,00 g
dest. Wasser zum Auffüllen auf 100,00 ml

Das Nipagin® die Citronensäure werden in 20 ml destilliertem Wasser gelöst. Zu der Lösung gibt man in kleinen Portionen, unter intensivem Rühren das Carbopol® und läßt die Lösung dann 10-12 Stunden lang stehen. Anschließend werden die mit 1 ml destilliertem Wasser bereitete Lösung des Natriumhydroxyds, dann die wäßrige Lösung des Sorbits und schließlich die äthanolische Lösung des Himbeeraromas unter Rühren zugegeben. Zu der so bereiteten Trägersubstanz gibt man in kleinen Portionen den Wirkstoff und homogenisiert das Ganze mit einem Mixer. Die Suspension wird mit destilliertem Wasser auf 100 ml aufgefüllt und in einer Kolloidmühle homogenisiert. Wirkstoff: 1-(2-Methoxyphenyl)-1-{4-[3-(4-methyl-piperazin-1-yl)-propoxy]-phenyl}-propan-1-ol.

**Patentansprüche**

für die Vertragsstaaten: BE, CH,DE, FR, GB, IT, LI, LU, NL, SE

1). Substituierte 1-{[3-(amino)-propoxy]-phenyl}-1-{phenyl}-propan-1-ole der allgemeinen Formel

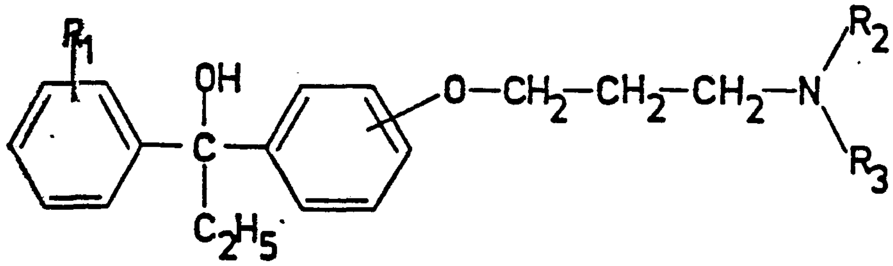

I,

worin

$R_1$ für ein Halogenatom, einen Trihalogenmethylrest oder einen Alkoxy- oder Alkylrest mit je 1 bis 3 Kohlenstoffatom(en) steht,

$R_2$ einen Alkylrest mit 1 bis 3 Kohlenstoffatom(en) bedeutet und

$R_3$ einen Cycloalkylrest mit 3 bis 6 Kohlenstoffatomen darstellt oder

$R_2$ und $R_3$ zusammen mit dem Stickstoffatom, an welches sie gebunden sind, einen, gegebenenfalls durch 1 oder mehr Alkylrest(e) mit 1 bis 4 Kohlenstoffatom(en) oder Benzylrest(e) substituierten und/oder gegebenenfalls als weiteres Heteroatom ein Sauerstoffatom oder ein weiteres Stickstoffatom aufweisenden, heterocyclischen Rest mit höchstens 8 Gliedern darstellt, sowie ihre Säureadditionssalze und quaternären Salze.

2.) 1-{[3-(Amino)-propoxy]-phenyl}-1-{phenyl}-propan-1-ole nach Anspruch 1, dadurch gekennzeichnet, daß das Halogen, für welches $R_1$ stehen kann, Chlor oder Fluor ist.

3.) 1-{[3-(Amino)-propoxy]-phenyl}-1-{ phenyl}-propan-1-ole nach Anspruch 1, dadurch gekennzeichnet, daß der Trihalogenmethylrest, für den $R_1$ stehen kann, ein Trifluormethylrest oder Trichlormethylrest ist.

4.) 1-{[3-(Amino)-propoxy]-phenyl}-1-{phenyl} -propan-1-ole nach Anspruch 1, dadurch gekennzeichnet, daß der Alkoxyrest, für den $R_1$ stehen kann und/oder der beziehungsweise die Alkylrest(e), für den beziehungsweise die $R_1$ und/oder $R_2$ stehen kann beziehungsweise können, ein solcher beziehungsweise solche mit 1 oder 2, insbesondere 1, Kohlenstoffatom(en) ist beziehungsweise sind.

5.) 1-{[3-(Amino)-propoxy]-phenyl}-1-{phenyl}-propan-1-ole nach Anspruch 1 bis 4, dadurch gekennzeichnet, daß der Cycloalkylrest, für den $R_3$ stehen kann, ein solcher mit 5 oder 6, insbesondere 6, Kohlenstoffatomen ist.

6.) 1-{[3-(Amino)-propoxy]-phenyl}-1-{phenyl}-propan-1-ole nach Anspruch 1 bis 4, dadurch gekennzeichnet, daß der heterocyclische Rest, den $R_2$ und $R_3$ zusammen mit dem Stickstoffatom, an welches sie gebunden sind, darstellen können, ein solcher mit 3 bis 8, insbesondere 5 oder 6, ganz besonders 6, Kohlenstoffatomen, vor allem ein Morpholinyl-, Piperazinyl- oder Imidazolylrest, ist.

7.) 1-{[3-(Amino)-propoxy]-phenyl}-1-{phenyl}-propan-1-ole nach Anspruch 1 bis 4 oder 6, dadurch gekennzeichnet, daß der beziehungsweise die Alkylrest(e), durch welchen beziehungsweise welche der heterocyclische Rest, den $R_2$ und $R_3$ zusammen mit dem Stickstoffatom, an welches sie gebunden sind, darstellen können, substituiert sein kann, ein solcher beziehungsweise solche mit 1 bis 3, insbesondere 1 oder 2, ganz besonders 1, Kohlenstoffatom(en) ist beziehungsweise sind.

8.) Die 1-{[3-(Amino)-propoxy]-phenyl}-1-{phenyl}-propan-1-ole 1-(4-Chlorphenyl)-1 -{4-[3-(4-methyl-piperazin-1-yl)-propoxy]-phenyl}-propan-1-ol,-1-(3-Trifluormethylphenyl)-1-{4-[3-(morpholin-1-yl)-propoxy]-phenyl}-propan-1-ol, 1-(4-Fluorphenyl)-1-{4-[3-(4-benzyl-piperazin-1-yl)-propoxy]-phenyl}-propan-1-ol, 1-(3-Chlor-phenyl)-1-{4-[3-(4-benzyl-piperazin-1-yl)-propoxy]-phenyl}-propan-1-ol, 1-(3-Trifluormethylphenyl)-1-{4-[3-(4-methyl- piperazin-1-yl)-propoxy]-phenyl}-propan-1-ol, 1-(2-Methoxy-phenyl)-1-{4-[3-(4-methyl-piperazin-1-yl)-propoxy]-phenyl}-propan-1-ol, 1-(4-Chlorphenyl)-1-{4-[3-(4-benzyl-piperazin-1-yl)-propoxy]-phenyl}-propan-1-ol, 1-(3-Chlorphenyl)-1-{4-[3-(4-methyl-piperazin-1-yl)-propoxy]-phenyl}-propan-1-ol, 1-(2-Methoxyphenyl)-1-{4-[3-(4-benzyl-piperazin-1-yl)-propoxy]-phenyl}-propan-1-ol, 1-(2-Methoxyphenyl)-1-{4-[3-(2-methyl-imidazol-1-yl)-propoxy]-phenyl}-propan-1-ol,1-(2-Methoxyphenyl)-1-{4-[3-(imidazol-1-yl)-propoxy]-phenyl}-propan-1-ol und 1-(3-Trifluormethylphenyl)-1-{4-[3-(N-methyl-N-cyolohexyl-amino)-propoxy]-phenyl}-propan-1-ol sowie ihre Salze.

9.) Verfahren zur Herstellung der Verbindungen nach Anspruch 1 bis 8, dadurch gekennzeichnet, daß man

a) am Benzolring 3-(amino)-propoxysubstituierte Propiophenone der allgemeinen Formel

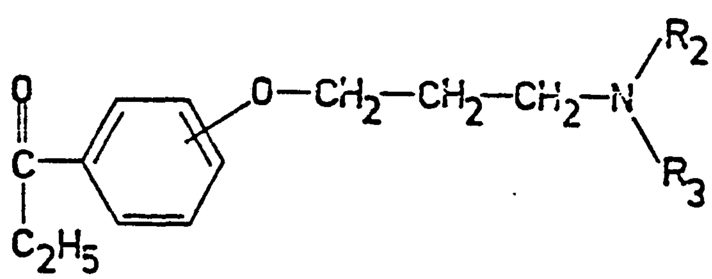

II,

worin $R_2$ und $R_3$ wie in den Ansprüchen 1 oder 4 bis 7 festgelegt sind, mit eine substituierte Phenylgruppe aufweisenden metallorganischen Verbindungen der allgemeinen Formel

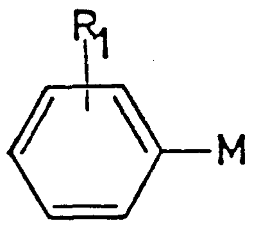

III ,

worin
$R_1$ wie in den Ansprüchen 1 bis 4 festgelegt ist und
M für ein Alkalimetallatom, vorzugsweise Lithium-, Kalium- oder Natriumatom, oder einen Rest der Formel MgX mit X = Halogenatom steht,
unsetzt oder
b) substituierte 1-{[3-(Halogen)-propoxyl-phenyl}-1-{phenyl}-propan-1-ole der allgemeinen Formel

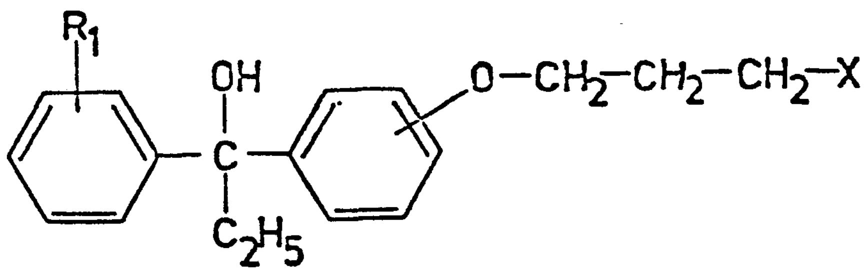

IV ,

worin
$R_1$ wie in den Ansprüchen 1 bis 4 f estgelegt ist und
X für ein Halogenatom steht,
mit sekundären Aminen der allgemeinen Formel

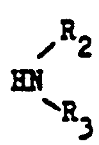

V ,

worin $R_2$ und $R_3$ wie in den Ansprüchen 1 oder 4 bis 7 festgelegt sind, vorzugsweise in Gegenwart von säurebindenden Mitteln, umsetzt oder
c) substituierte [3-(Amino) -propoxy]-benzophenone der allgemeinen Formel

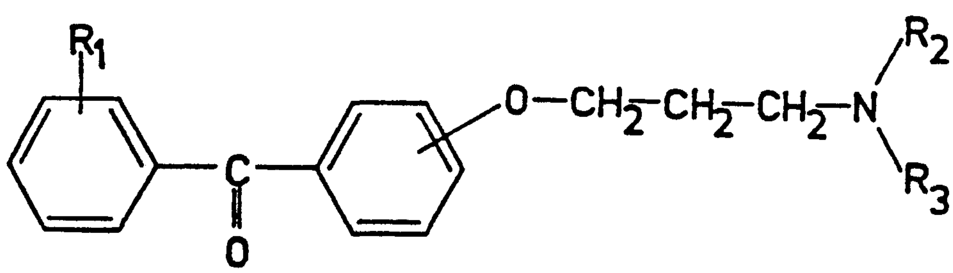

VI ,

worin $R_1$ $R_2$ und $R_3$ wie in den Ansprüchen 1 bis 7 festgelegt sind, mit eine Äthylgruppe aufweisenden metallorganischen Verbindungen, vorzugsweise Äthylmagnesiumhalogeniden oder Äthyllithium, umsetzt oder

d) am Benzolring substituierte Propiophenone der allgemeinen Formel

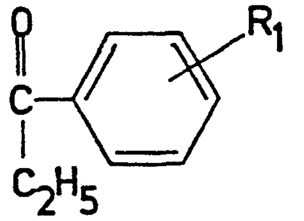

VII ,

worin $R_1$ wie in den Ansprüchen 1 bis 4 festgelegt ist, mit eine {[3-(Amino)-propoxy]-phenyl}-gruppe aufweisenden Grignard-Verbindungen der allgemeinen Formel

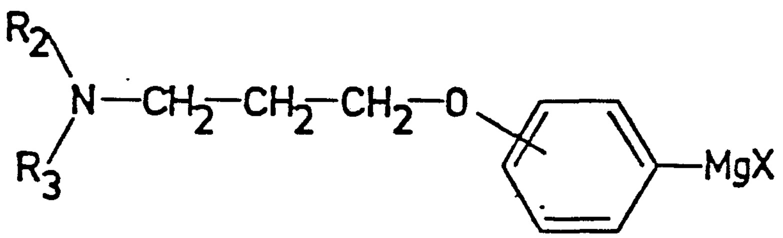

VIII ,

worin
$R_2$ und $R_3$ wie in den Ansprüchen 1 oder
4 bis 7 festgelegt sind und
X Halogen bedeutet,
umsetzt oder
e) substituierte 1-(Phenyl)-1-(hydroxyphenyl)-pro-pan-1-ole der allgemeinen Formel

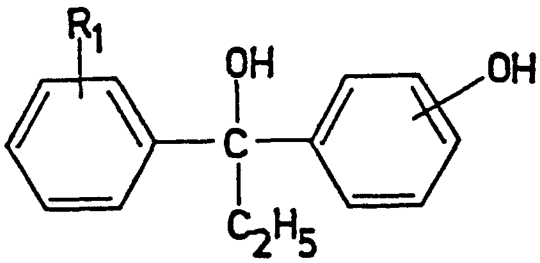

IX ,

worin $R_1$ wie in den Ansprüchen 1 bis 4 festgelegt ist, vorzugsweise in Form ihrer Alkaliphenolate oder quaternären Ammoniumphenolate, mit tertiären Aminen der allgemeinen Formel

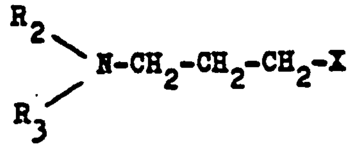

X ,

worin
$R_2$ und $R_3$ wie in den Ansprüchen 1 oder 4 bis 7 festgelegt sind und
X für einen Alkylsulfonyloxy-oder Arylsulfonyloxyrest oder ein Halogenatom steht,
oder mit deren Salzen, vorzugsweise in Gegenwart von säurebindenden Mitteln, umsetzt,
worauf man gegebenenfalls in an sich bekannter Weise die erhaltenen 1-{[3-(Amino)-propoxyl-phenyl}-1-{phenyl}-propan-1-ole der allgemeinen Formel I mit Säuren in Säureadditionssalze oder mit Quaternisierungsmitteln in quaternäre Ammoniumsalze überführt oder gegebenenfalls die erhaltenen Säureadditionssalze der 1-{[3-(Amino)-propoxyl-phenyl}-1-{phenyl}-propan-1-ole der allgemeinen Formel I in die entsprechenden freien Basen der allgemeinen Formel I und/oder in andere Säureadditionssalze oder quaternäre Ammoniumsalze überführt.

10.) Arzneimittel, gekennzeichnet durch einen Gehalt an 1 oder mehr Verbindung(en) nach Anspruch 1 bis 8 als Wirkstoff(en), zweckmäßigerweise zusammen mit 1 oder mehr üblichen pharmazeutischen Konfektionierungsmittel(n).

**Patentansprüche für den Vertragsstaat: AT**

1. Verfahren zur Herstellung von substituierten 1-{[3-(Amino) propoxy]-phenyl}-1-(phenyl}-propan-1-olen der allgemeinen Formel

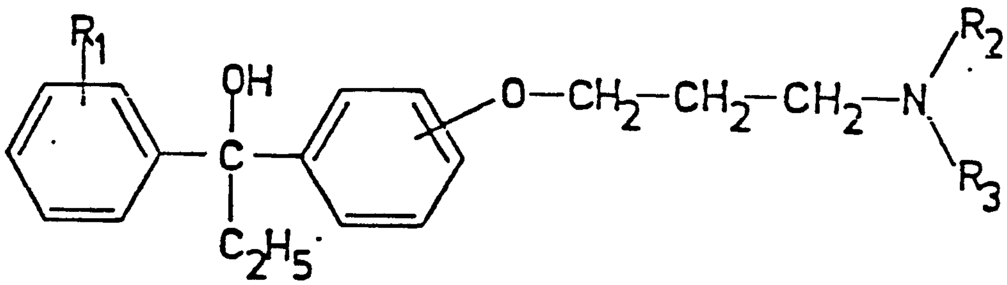

I ,

worin

$R_1$ für ein Halogenatom, einen Trihalogenmethylrest oder einen Alkoxy- oder Alkylrest mit je 1 bis 3 Kohlenstoffatom(en) steht,

$R_2$ einen Alkylrest mit 1 bis 3 Kohlenstoffatom(en) bedeutet und

$R_3$ einen Cycloalkylrest mit 3 bis 6 Kohlenstoffatomen darstellt oder

$R_2$ und $R_3$ zusammen mit dem Stickstoffatom, an welches sie gebunden sind, einen, gegebenenfalls durch 1 oder mehr Alkylrest(e) mit 1 bis 4 Kohlenstoffatom(en) oder Benzylrest(e) substituierten und/oder gegebenenfalls als weiteres Heteroatom ein Sauerstoffatom oder ein weiteres Stickstoffatom aufweisenden, heterocyclischen Rest mit höchstens 8 Gliedern darstellt sowie ihrer Säureadditionssalze und quaternären Salze, dadurch gekennzeichnet, daß man

a) am Benzolring 3-(amino)-propoxyßsubstituierte Propiophenone der allgemeinen Formel

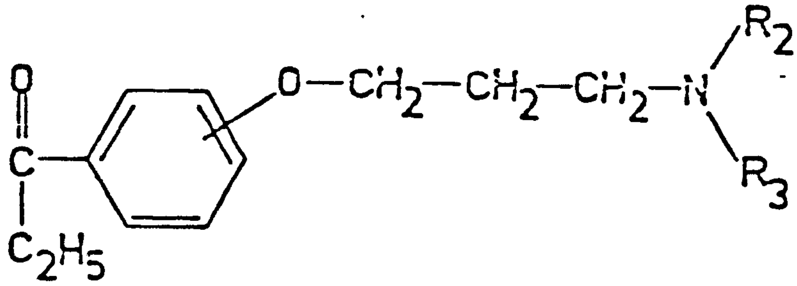

II ,

worin $R_2$ und $R_3$ die vorstehend angegebenen Bedeutungen besitzen, mit eine substituierte Phenylgruppe aufweisenden metallorganischen Verbindungen der allgemeinen Formel

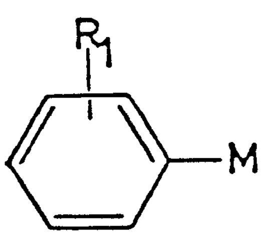

III ,

worin

$R_1$ die vorstehend angegebene Bedeutung besitzt und

M für ein Alkalimetallatom, vorzugsweise Lithium- Kalium- oder Natriumatom, oder einen Rest der Formel MgX mit X = Halogenatom steht,

umsetzt oder

b) substituierte 1-{[3-(Halogen)-propoxy]-phenyl}-1-{phenyl} -propan-1-ole der allgemeinen Formel

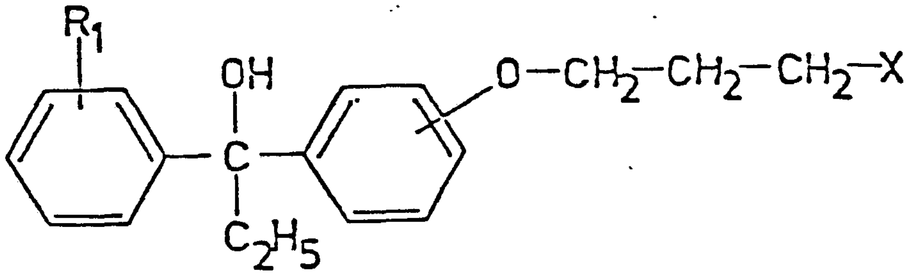

IV ,

worin
$R_1$ die vorstehend angegebene Bedeutung besitzt und
X für ein Halogenatom steht,
mit sekundären Aminen der allgemeinen Formel

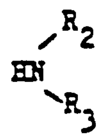

V ,

worin $R_2$ und $R_3$ die vorstehend angegebenen Bedeutungen besitzen, vorzugsweise in Gegenwart von säurebindenden Mitteln umsetzt oder

c) substituiere [3-(Amino)-propoxy]-benzophenone der allgemeinen Formel

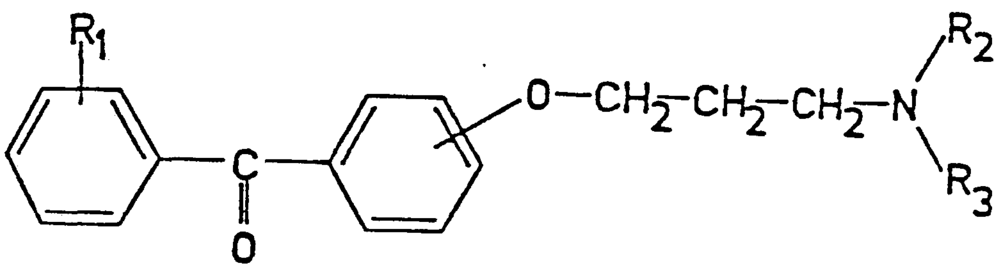

VI ,

worin $R_1$, $R_2$ und $R_3$ die vorstehend angegebenen Bedeutungen besitzen, mit eine Äthylgruppe aufweisenden metallorganischen Verbindungen, vorzugsweise Äthylmagnesiumhalogeniden oder Äthyllithium, umsetzt oder

d) am Benzolring substituierte Propiophenone der allgemeinen Formel

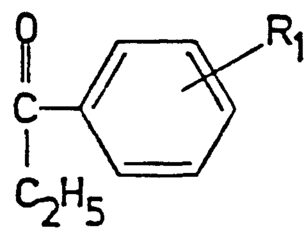

VII ,

worin $R_1$ die vorstehend angegebene Bedeutung besitzt, mit eine {[3-(Amino)-propoxy]-phenyl}-gruppe aufweisenden Grignard-Verbindungen der allgemeinen Formel

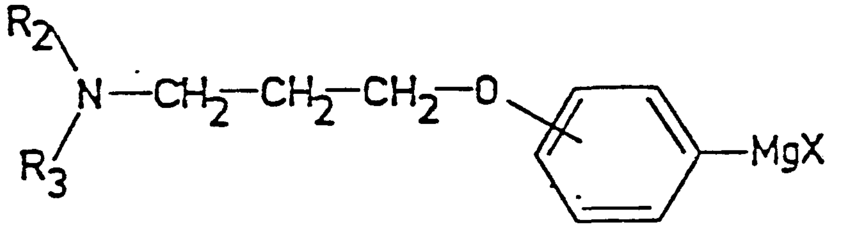

VIII. ,

worin
$R_2$ und $R_3$ die vorstehend angegebenen Bedeutungen besitzen und
X Halogen bedeutet,
umsetzt oder

e) substituierte 1-(Phenyl)-1-(hydroxyphenyl) pro-pan-1-ole der allgemeinen Formel

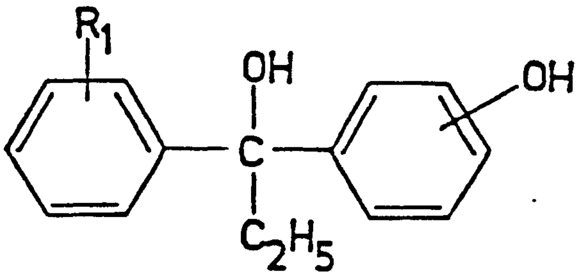

II ,

worin $R_1$ die vorstehend angegebene Bedeutung bebesitzt, vorzugsweise in Form ihrer Alkaliphenolate oder quaternären Ammoniumphenolate, mit tertiären Aminen der allgemeinen Formel

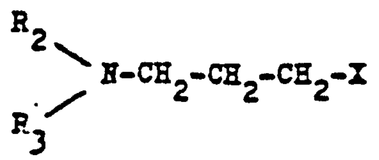

I ,

worin
$R_2$ und $R_3$ die vorstehend angegebene Bedeutungen besitzen und
X für einen Alkylsulfonyloxy-oder Arylsulfonyloxyrest oder ein Halogenatom steht,
oder mit deren Salzen, vorzugsweise in Gegenwart von säurebindenden Mitteln, umsetzt,
worauf man gegebenenfalls in an sich bekannter Weise die erhaltenen 1-{[3-(Amino)-propoxy]-phenyl}-1-{phenyl}-propan-1-ole der allgemeinen Formel I mit Säuren in Säureadditionssalze oder mit Quaternisierungsmitteln in quaternäre Ammoniumsalze überführt oder gegebenenfalls die erhaltenen Säureadditionssalze der 1-{[3-(Amino)-propoxy]-phenyl}-1-{phenyl}-propan-1-ole der allgemeinen Formel I in die entsprechenden freien Basen der allgemeinen Formel I und/oder in andere Säureadditionssalze oder quaternäre Ammoniunsalze überführt.

2. Verfahren nach Anspruch 1, dadurch gekennzeichnet, daß das Halogen, für welches $R_1$ stehen kann Chlor oder Fluor ist.

3. Verfahren nach Anspruch 1, dadurch gekennzeichnet, daß der Trihalogenmethylrest, für den $R_1$ stehen kann, ein Trifluormethylrest oder Trichlormethylrest ist.

4. Verfahren nach Anspruch 1, dadurch gekennzeichnet, daß der Alkoxyrest, für den $R_1$ stehen kann und/oder der beziehungsweise die Alkylrest(e), für den beziehungsweise die $R_1$ und/oder $R_2$ stehen kann, beziehungsweise können, ein solcher beziehungsweise solche mit 1 oder 2, insbesondere 1, Kohlenstoffatom(en) ist beziehungsweise sind.

5. Verfahren nach Anspruch 1 bis 4, dadurch gekennzeichnet, daß der Cycloalkylrest, für den $R_3$ stehen kann, ein solcher mit 5 oder 6, insbesondere 6, Kohlenstoffatomen ist.

6. Verfahren nach Anspruch 1 bis 4, dadurch gekennzeichnet, daß der heterocyclische Rest, den $R_2$ und $R_3$ zusammen mit dem Stickstoffatom, an welches sie gebunden sind, darstellen können, ein solcher mit 3 bis 8, insbesondere 5 oder 6, ganz besonders 6, Kohlenstoffatomen, vor allem ein Morpholinyl-, Piperazinyl- oder Imidazolylrest, ist.

7. Verfahren nach Anspruch 1 bis 4 oder 6, dadurch gekennzeichnet, daß der beziehungsweise die Alkylrest(e), durch welchen beziehungsweise welche der heterocyclische Rest, den $R_2$ und $R_3$ zusammen mit dem Stickstoffatom, an welches sie gebunden sind, darstellen können, substituiert sein kann, ein solcher beziehungsweise solche mit 1 bis 3, insbesondere 1 oder 2, ganz besonders 1, Kohlenstoffatom(en) ist beziehungsweise sind.

8. Verfahren nach Anspruch 1 bis 7, dadurch gekennzeichnet, daß -{4-[3-(4-methyl-piparamin-1-yl)-propoxy]-phenyl}-propan-1-ol, 1-(3-Trifluormethylphenyl)-1-{4-[3-(morpholin-1-yl)-propoxy]-phenyl}-propan-1-ol, 1-(4-Fluorphenyl)-1-{4-[3-(4-benzyl-piperazin-1-yl)-propoxy]-phenyl}-propan-1-ol, 1-(3-Chlor-phenyl)-1-{4-[3-(4-benzyl-piperazin-1-yl)-propoxy]-phenyl}-propan-1-ol, 1-(3-Triflurmethylphenyl)-1-{4-[3-(4-methyl- piperazin-1-yl)-propony]-phenyl}-propan-1-ol, 1-(2-Methoxy-phenyl)-1-{4-[3-(4-methyl-piperazin-1-yl)-propoxy]-phenyl}-propan-1-ol, 1-(4-Chlorphenyl)-1-{4-[3-(4-benzyl-piperazin-1-yl)-propoxy]-phenyl}-propan-1-ol, 1-(3-Chlorphenyl)-1-{4-[3-(4-methyl-piperazin-1-yl)-propoxy]-phenyl}-propan-1-ol, 1-(2-Methoxyphenyl)-1-{4-[3-(4-benzyl-piperazin-1-yl)-propony ]-phenyl}-propan-1-ol, 1-(2-Methoxyphenyl)-1-{4-[3-(2-methyl-imidazol-1-yl)-propoxy]-phenyl}-propan-1-ol, 1-(2-Methoxyphenyl)-1-{4-[3-(imidazol-1-yl)-propoxy]-phenyl}-propan-1-ol und 1-(3-Trifiourmethylphenyl)-1-{4-[3-(N-methyl-N-cyclohexyl-amino)-propoxy]-phenyl}-propan-1-ol sowie ihre Salze hergestellt werden.

**Claims**

for the contracting states: BE, CH, DE, FR, GB, IT, LI, LU, NL, SE

1. Substituted 1-{[3-(amino)-propoxy]-phenyl}-1-{phenyl}-propane-1-oles of the general formula

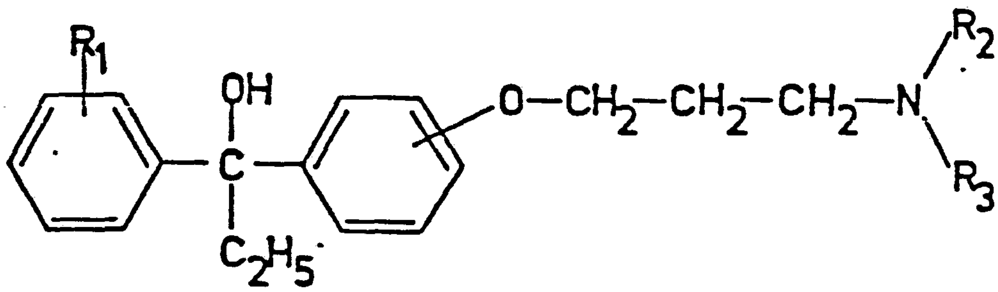

I ,

wherein

$R_1$ is a halogen atom, a trihalomethyl radical or an alkoxy- or alkyl radical having each 1 to 3 carbon atom (s),
$R_2$ is an alkyl radical having 1 to 3 carbon atom (s), and
$R_3$ is a cycloalkyl radical having 3 to 6 carbon atoms, or
$R_2$ and $R_3$ together with the nitrogen atom with which they are attached are forming a heterocyclic radical having at most 8 members being optionally substituted by 1 or more alkyl radical(s) having 1 to 4 carbon atom(s) or benzyl radical(s) and/or optionally having as additional hetero atom an oxygen atom or an additional nitrogen atom, and their acid addition salts and quaternary salts.

2. 1 - {[3-(Amino)-propoxy]-phenyl}-1-{phenyl}-propane-1-oles according to claim 1, characterized in that the halogen for which $R_1$ can stand is chlorine or fluorine.

3 - 1 {[3-(Amino)-propoxy]-phenyl}-1-{phenyl}-propane-1-oles according to claim 1, characterized in that the trihalomethyl radical for which $R_1$ can stand is a trifluoromethyl radical or a trichloromethyl radical.

4. 1 {[3-(Amino)-propoxy]-phenyl}-1-{phenyl}-propane-1-oles according to claim 1, characterized in that the alkoxy radical for which $R_1$ can stand and/or the alkyl radical(s), respectively, for which the substituents $R_1$ and/or $R_2$ can stand, is a such one and are those ones, respectively having 1 or 2, especially 1, carbon atom(s).

5. 1-{[3-(Amino)-propoxy]-phenyl}-1-{phenyl}-propane-1-oles according to claim 1 to 4, characterized in that the cycloalkyl radical for which $R_3$ can stand is a such one having 5 or 6, especially 6, carbon atoms.

6. 1-{[3-(Amino)-propoxy]-phenyl}-1-{phenyl}-propane-1-oles according to claims 1 to 4, characterized in that the heterocyclic radical which $R_2$ and $R_3$ together with the nitrogen atom being connected therewith can form is a such one having 3 to 8, especially 5 or 6, more preferably 6, carbon atoms, especially a morpholinyl-, piperazinyl- or imidazolyl radical.

7 1-{[3-(Amino)-propoxy]-phenyl}-1-{phenyl}-propane-1-oles according to claim 1 to 4 or 6, characterized in that the alkyl radical(s) by which the heterocyclic radical which is formed by $R_2$ and $R_3$ together with the nitrogen atom with which they are connected can form, can be substituted is a such one and are those ones, respecitvely having 1 to 3, especially 1 or 2, more preferably 1, carbon atom(s).

8. The 1-{[3-(Amino)-propoxy]-phenyl}-1-{phenyl}-propane-1-oles 1-(4-chlorophenyl)-1-{4-[3-4-methyl-piperazin o-1-yl)-propoxy]-phenyl}-propane-1-ol, 1-(3-trifluoromethylphenyl)-1-{4-[3-(morpholino-1-yl)-propoxy]-phenyl}-propane-1-ol, 1-(4-fluorophenyl)-1-{4-[3-(4-benzylpiperazino-1-yl)-propoxy]-phenyl}-propane-1-ol, 1-(3-chlorophenyl)-1-{4-[3-(4-benzyl-piperazino-1-yl)-propoxy]-phenyl}-propane-1-ol, 1-(3-trifluoromethylphenyl)-1-{4-[3-(4-methylpipera zino-1-yl)-propoxy]-phenyl}-propane-1-ol, 1-(2-methoxyphenyl)-1-{4[3(4-methyl-piperazino-1-yl)-propoxy]-phenyl}-propane-1-ol, 1-(4-chlorophenyl)-1-{4-[3-(4-benzyl-piperazino-1-yl)-propoxy]-phenyl}-propane-1-ol, 1-(3-chlorophenyl)-1-{4-[3-(4-methyl-piperazino-1-yl)-prop-oxy]-phenyl}-propane-1-ol, 1-(2-methoxyphenyl)-1-{4-[3-(4-benzyl-piperazino-1-yl)-propoxy]-phenyl}-propane-1-ol, 1-(2-methoxyphenyl)-1-{4-[3-(2-methyl-imidazolo-1-yl)-propoxy]-phenyl}propane-1-ol, 1-(2-methoxyphenyl)-1-{4-[3-(imidazolo-1-yl)-propoxy]-phenyl}-propane-1-ol and 1-(3-trifluoromethylphenyl)-1-{4-[3-(N-methyl-N-cyclohexyl-amino)-propoxy]-phenyl}-propane-1-ol, and their salts.

9. A process for preparing the compounds according to claim 1 to 8, characterized in that

a) at the benzene ring 3- (amino)-propoxy- substituted propiophenones of the general formula

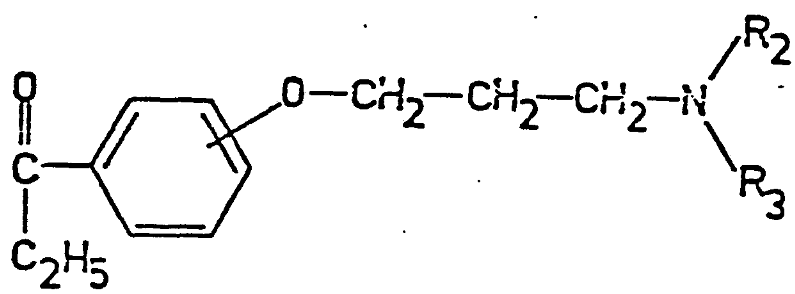

II ,

wherein $R_2$ and $R_3$ are defined as in the claims 1 or 4 to 7, are reacted with a organometallic compound having a substituted phenyl group of the general formula

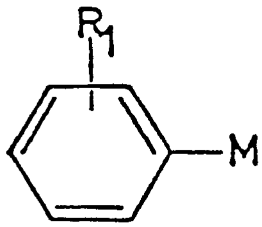

III ,

wherein

$R_1$ is defined as in the claims 1 to 4, and M is an alkali metal atom, preferably lithium-, potassium- or sodium atom, or a radical of the formula MgX, X being a halogen atom, or

b) substituted 1-{[3-(halogen)-propoxy]-phenyl}-1-{phenyl}-propane-1-oles of the general formula

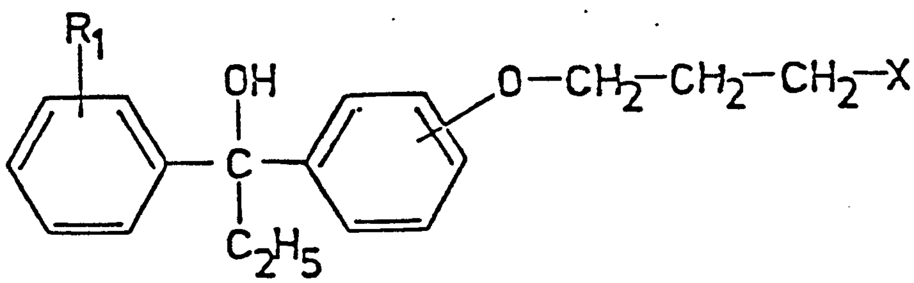

IV ,

wherein

$R_1$ is defined as in the claims 1 to 4, and

X is a halogen atom,

are reacted with secondary amines of the general formula

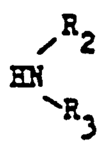

V ,

wherein $R_2$ and $R_3$ are defined as in the claims 1 or 4 to 7, preferably in the presence of acid binding agents, or

c) substituted [3- (amino) -propoxy] -benzophenones of the general formula

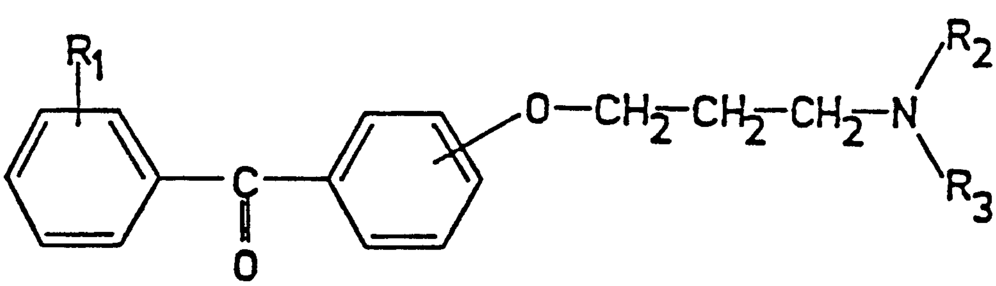

VI ,

wherein $R_1$, $R_2$ and $R_3$ are as defined as in the claims 1 to 7, are reacted with organometallic compounds having an ethyl group, preferably ethyl magnesium halides or ethyl lithium, or

d) at the benzene substituted propiophenones of the general formula

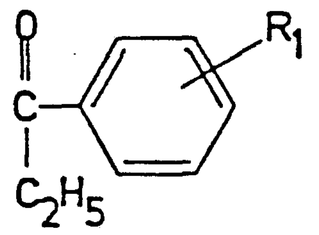

VII ,

wherein $R_1$ is defined as in the claims 1 to 4, are reacted with a Grignard-compound having a {[3- (amino) -propoxy] -phenyl} -group of the general formula

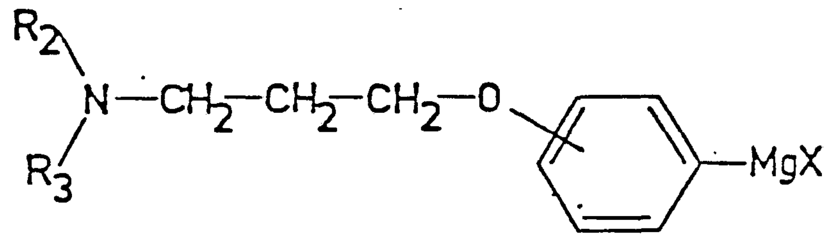

VIII ,

wherein
$R_2$ and $R_3$ are as defined in the claims 1 or 4 to 7, and
X is halogen, or
e) substituted 1 -(phenyl) -1-(hydroxyphenyl)-propane-1-oles of the general formula

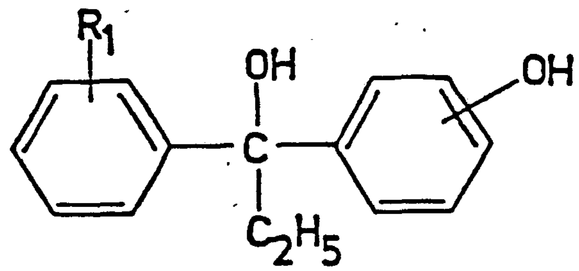

IX ,

wherein $R_1$ is as defined in claims 1 to 4, preferably in form of their alkali phenolates or quaternary ammonium phenolates, are reacted with tertiary amines of the general formula

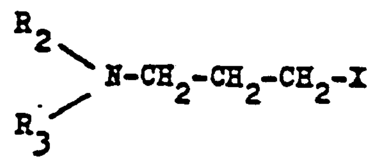

I ,

wherein
$R_2$ and $R_3$ are as defined in claims 1 or 4 to 7, and
X is an alkylsulfonyloxy- or arylsulfonyloxy radical or a halogen atom,
or with their salts, preferably in the presence of acid binding agents, and
subsequently optionally in a manner known per se the obtained 1-{[3-(amino)-propoxy]-phenyl}-1-{phenyl}-pro-pane-1-oles of the general formula I are converted with acids into acid addition salts or with quaternizing agents into quaternary ammonium salts, or optionally the obtained acid addition salts of the 1-{[3-(amino)-propoxy]-phenyl}-1-{phenyl}-propane-1-oles of the general formula I are converted into the corresponding free bases of the general formula I and/or into different acid addition salts or quaternary ammonium salts.

10. Pharmaceutical compositions, characterized by a content of 1 or more compound(s) according to claim 1 to 8 as active ingredient(s), preferably together with 1 or more conventional pharmaceutical auxiliary agent (s).

**Claims**

for the contracting state: AT

1. A process for preparing substituted 1-{[3-(amino)-propoxy]-phenyl}-1-{phenyl}-propane-1-oles of the

general formula

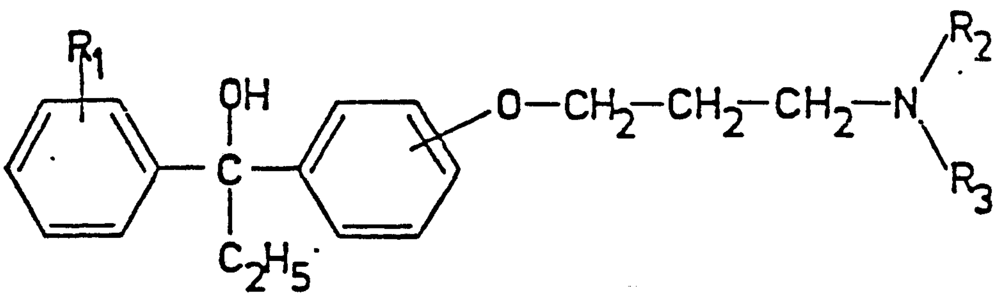

I ,

wherein

$R_1$ is a halogen atom, a trihalomethyl radical or an alkoxy- or alkyl radical having each 1 to 3 carbon atom(s),
$R_2$ is an alkyl radical having 1 to 3 carbon atom (s), and
$R_3$ is a cycloalkyl radical having 3 to 6 carbon atoms, or
$R_2$ and $R_3$ together with the nitrogen atom with which they are attached are forming a heterocyclic radical having at most 8 members being optionally substituted by 1 or more alkyl radical(s) having 1 to 4 carbon atom (s) or benzyl radical(s) and/or optionally having as additional hetero atom an oxygen atom or an additional nitrogen atom,
and their acid addition salts and quaternary salts, characterized in that
a) at the benzene ring 3- (amino)-propoxy substituted propiophenones of the general formula

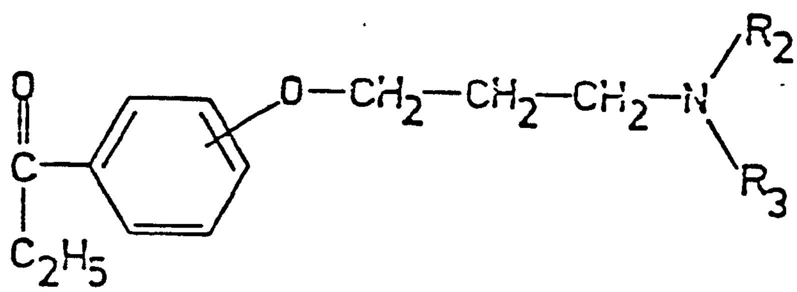

II ,

wherein $R_2$ and $R_3$ are defined as in the claims 1 or 4 to 7, are reacted with an organometallic compound having a substituted phenyl group of the general formula

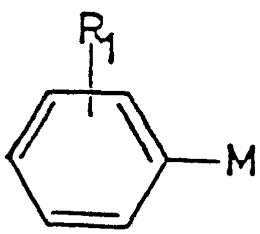

III ,

wherein
$R_1$ is defined as in the claims 1 to 4, and M is an alkali metal atom, preferably lithium-, potassium- or sodium atom, or a radical of the formula MgX, X being a halogen atom, or
b) substituted 1-{[3-(halogen)-propoxy]-phenyl}-1-{phenyl}-propane-1-oles of the general formula

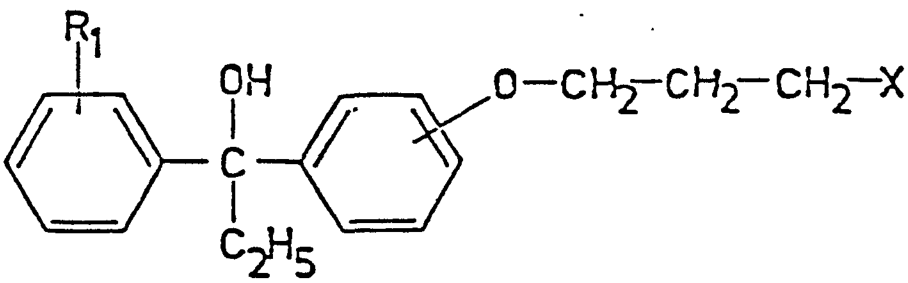

IV ,

wherein
$R_1$ is defined as in the claims 1 to 4, and
X is a halogen atom,
are reacted with secondary amines of the general formula

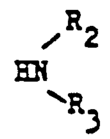

V ,

wherein $R_2$ and $R_3$ are defined as in the claims 1 or 4 to 7, preferably in the presence of acid binding agents, or

c) substituted [3-(amino)-propoxy]-benzophenones of the general formula

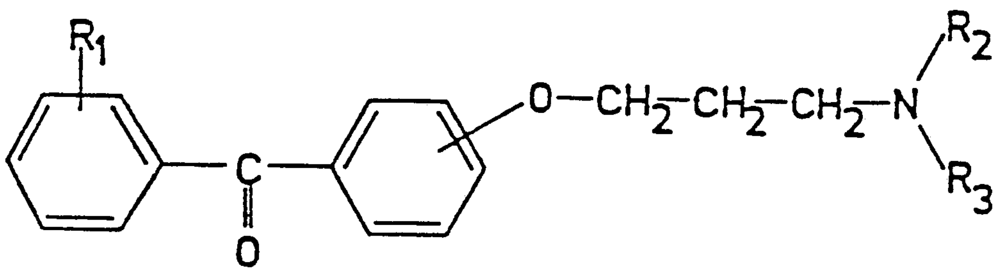

VI ,

wherein $R_1$, $R_2$ and $R_3$ are as defined as in the claims 1 to 7 are reacted with organometallic compounds having an ethyl group preferably ethyl magnesium halides or ethyl lithium, or

d) at the benzene substituted propiophenones of the general formula

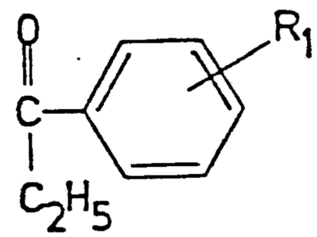

VII ,

wherein $R_1$ is defined as in the claims 1 to 4, are reacted with a Grignard-compound having a {[3-(amino)-propoxy]-phenyl}-group of the general formula

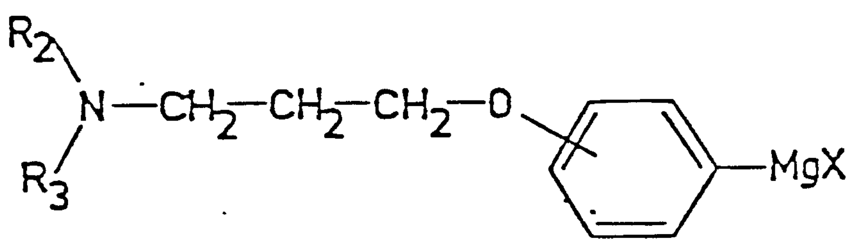

VIII ,

wherein

$R_2$ and $R_3$ are as defined in the claims 1 or 4 to 7, and

X is halogen, or

e) substituted 1 - (phenyl) -1 - (hydroxyphenyl) -propane-1-oles of the general formula

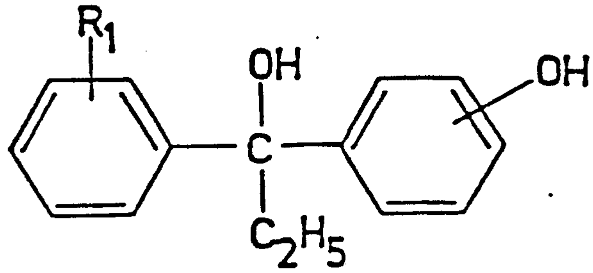

IX ,

wherein $R_1$ has the above meaning, preferably in form of their alkali phenolates or quaternary ammonium phenolates, are reacted with tertiary amines of the general formula

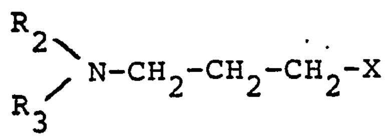 X,

wherein

$R_2$ and $R_3$ are as defined in claims 1 or 4 to 7, and

X is an alkylsulfonyloxy- or arylsulfonyloxy radical or a halogen atom, or with their salts, preferably in the presence of acid binding agents, and

subsequently optionally in a manner known per se the obtained 1-{(3-(amino)-propoxy]-phenyl}-1-{phenyl}-pro-pane-1-oles of the general formula I are converted with acids into acid addition salts or with quaternizing agents into quaternary ammonium salts, or optionally the obtained acid addition salts of the 1-{[3-(amino)-propoxy] -phenyl{-phenyl}-propane-1-oles of the general formula I are converted into the corresponding free bases of the general formula I and/or into different acid addition salts or quaternary ammonium salts.

2. A process according to claim 1, characterized in that the halogen for which $R_1$ can stand is chlorine or fluorine.

3. A process according to claim 1, characterized in that the trihalomethyl radical for which $R_1$ can stand is a trifluoromethyl radical or a trichloromethyl radical.

4. A process according to claim 1, characterized in that the alkoxy radical for which $R_1$ can stand and/or the alkyl radical(s), respectively for which the substituents $R_1$ and/or $R_2$ can stand is a such one and are those ones, respectively having 1 or 2, especially 1, carbon atom(s).

5. A process according to claim 1 to 4, characterized in that the cycloalkyl radical for which $R_3$ can stand is a such one having 5 or 6, especially 6, carbon atoms.

6. A process according to claim 1 to 4, characterized in that the heterocyclic radical which $R_2$ and $R_3$ together with the nitrogen atom with which they are connected can form, is a such one having 3 to 8, especially 5 or 6, more preferably 6, carbon atoms, especially a morpholinyl-, piperazinyl- or imidazolyl radical.

7. A process according to claim 1 to 4 or 6, characterized in that the alkyl radical(s) by which the heterocyclic radical which is formed by $R_2$ and $R_3$ together with the nitrogen atom with which they are connected, can be substituted is a such one and are those ones, respectively having 1 to 3, especially 1 or 2, more preferably 1, carbon atom(s).

8. A process according to claim 1 to 7, characterized in that 1-(4-chlorophenyl)-1-{4-[3-(4-methylpiperazino-1-yl)-propoxy]-phenyl)-propane-1-ol, 1-(3-trifluoromethylphenyl)-1-{4[3-(morpholino-1-yl)-propoxy]-phenyl}-propane-1-ol, 1-(4-fluorophenyl)-1-{4-[3-(4-metylpiperazino-1-yl)-propoxy]-phenyl}-propane-1-ol, 1-(3-chlorophenyl)-1-{4-[3-(4-benzylpiperazino-1-yl)-propoxy]})-phenyl}-propane-1-ol, 1-(3-trifluoromethylphenyl)-1-{4-]3-(4-methylpipera-zino-1-yl)-propoxy]-phenyl}-propane-1-ol, 1-(2-methoxy-phenyl)-1-{4-[3-(4-methylpiperazino-1-yl)-propoxy]-phe-nyl}-propane-1-ol, 1-(4-chlorophenyl)-1-}4-[3-(4-benzyl-piperazino-1-yl)-propoxy]-phenyl}-propane-1-ol, 1-(3-chlorophenyl)-1-{4-(3-(4-methyl-piperazino-1-yl)-prop-oxy]-phenyl}-propane-1-ol, 1-(2-methoxyphenyl)-1{4-[3-(4-benzyl-piperazino-1-yl)-propoxy]-phenyl}-propane-1-ol, 1-(2-methoxyphenyl)-1-{4-[3-(2-methyl-imidazolo-1-yl)-propoxy]-phenyl}-propane-1-ol, 1-(2-methoxyphenyl)-1-{4-[3-(imidazolo-1-yl)-propoxy]-phenyl}-propane-1-ol and 1-(3-trifluoromethylphenyl)-1-}4-[3-(N-methyl-N-cyclohexyl-amino)-propoxy]-phenyl}-propane-1-ol, and their salts are prepared.

## Revendications

pour les Etats contractants: BE, CH, DE, FR, GB, IT, LI, LU, NL, SE

1°) 1-{[3(amino)-propoxy]-phényl}-1-{phényl}-propan-1-ols substitués répondant à la formule générale

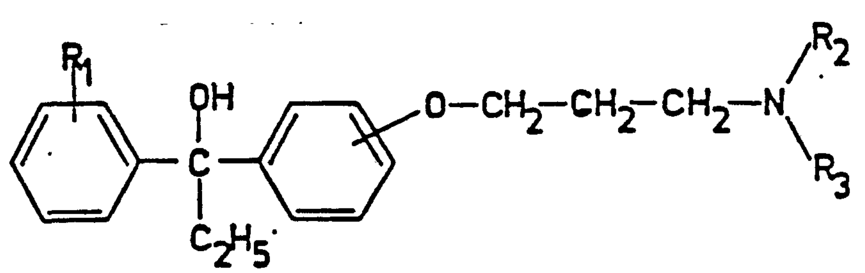 I ,

dans laquelle

$R_1$ représente un atome d'halogène, un reste trihalométhyle ou un reste alcoxy ou alkyle comportant dans chaque cas 1 à 3 atome(s) de carbone,

$R_2$ représente un reste alkyle comportant 1 à 3 atome(s) de carbone, et

$R_3$ représente un reste cycloalkyle comportant 3 à 6 atomes de carbone, ou

$R_2$ et $R_3$ représentent, avec l'atome d'azote auquel ils sont liés, un reste hétérocyclique comportant au plus 8 maillons, éventuellement substitué par 1 ou plusieurs reste(s) alkyle comportant 1 à 4 atome(s) de carbone ou reste(s) benzylique(s) et/ou comportant éventuellement, comme autre hétéroatome, un atome d'oxygène ou un autre atome d'azote,

ainsi que leurs sels d'addition avec des acides et sels quaternaires.

2°) 1-{[3-(amino)-propoxy]-phényl}-1-{phényl}-propan-1-ols selon la revendication 1, caractérisés en ce que l'halogène que peut représenter $R_1$ est le chlore ou le fluor.

3°) 1-{[3-(amino)-propoxy]-phényl}-1-{phényl}-propan-1-ols selon la revendication 1, caractérisés en ce que le reste trihalométhyle que peut représenter $R_1$ est un reste trifluorométhyle ou trichlorométhyle.

4°) 1-{[3-(amino)-propoxy]-phényl}-1-{phényl}-propan-1-ols selon la revendication 1, caractérisés en ce que le reste alcoxy que peut représenter $R_1$ et[ou le ou les reste(s) alkyle que peut ou peuvent représenter $R_1$ et/ou $R_2$ est ou sont un ou des reste(s) comportant 1 ou 2, en particulier 1 atome(s) de carbone.

5°) 1-{[3-(amino)-propoxy]-phényl}-1-{phényl}-propan-1-ols selon les revendications 1 à 4, caractérisés en ce que le reste cycloalkyle que peut représenter $R_3$ est un reste comportant 5 ou 6, en particulier 6, atomes de carbone.

6°) 1-{[3-(amino)-propoxy]-phényl}-1-{phényl}-propan-1-ols selon les revendications 1 à 4, caractérisés en ce que le reste hétérocyclique que $R_2$ et $R_3$ peuvent représenter avec l'atome d'azote auquel ils sont liés est un reste comportant 3 à 8, en particulier 5 ou 6, tout particulièrement 6, atomes de carbone, surtout un reste morpholinyle, pipérazinyle ou imidazolyle.

7°) 1-{[3-(amino)-propoxy]-phényl}-1-{phényl}-propan-1-ols selon les revendications 1 à 4 ou 6, caractérisés en ce que le ou les reste(s) alkyle par lequel ou lesquels le reste hétérocyclique que $R_2$ et $R_3$ peuvent représenter avec l'atome d'azote auquel ils sont liés peut être substitué, est ou sont un ou des reste(s) comportant 1 à 3, en particulier 1 ou 2, tout particulièrement 1, atome(s) de carbone.

8°) Les 1-{[3-(amino)-propoxy]-phényl}-1-{phényl}-propan-1-ols suivants: le 1-(4-chlorophényl)-1-{4-[3-(4-méthyl-pipérazin-1-yl)-propoxy]-phényl}-propan-1-ol, le 1-(3-trifluorométhylphényl-1-{4-[3-(morpholin-1-yl)-propoxy]-phényl}-propan-1-ol, le 1-(4-fluorophényl)-1-{4-[3-(4-benzyl-pipérazin-1-yl)-propoxy]-phényl}-propan-1-ol, le 1-(3-chlorophényl)-1-{4-[3-(4-benzyl-pipérazin-1-yl)-propoxy]-phényl}-propan-1-ol, le 1-(3-trifluorométhylphényl)-1-{4-[3-(4-méthyl-pipérazin-1-yl)-propoxy]-phényl}-propan-1-ol, le 1-(2-méthoxyphényl)-1-{4-[3-(4-méthyl-pipérazin-1-yl)-propoxy]-phényl}-propan-1-ol), le 1-(4-chlorophényl)-1-{4-[3-(4-benzyl-pipérazin-1-yl)-propoxy]-phényl}-propan-1-ol, le 1-(3-chlorophényl)-1-{4-[3-(4-méthyl-pipérazin-1-yl)-propoxy]-phényl}-propan-1-ol, le 1-(2-méthoxyphényl)-1-{4-[3-(4-benzyl-pipérazin-1-yl)-propoxy[-phényl}-propan-1-ol, le 1-(2-méthoxyphényl)-1-{4-[3-(2-méthyl-imidazol-1-yl)-propoxy[-phényl}-propan-1-ol, le 1-(2-méthoxyphényl)-1-{4-[3-(imidazol-1-yl)-propoxy]-phényl}-propan-1-ol et le 1-{(3-trifluorométhylphényl)-1-}4-[3-(N-méthyl-N-cyclohexyl-amino)-propoxy]-phényl -propan-1-ol, ainsi que leurs sels.

9°) Procédé de préparation des composés selon les revendications 1 à 6, caractérisé en ce que

a) on fait réagir des propiophénones 3-(amino)-propoxysubstituées sur le noyau benzénique répondant à la formule générale

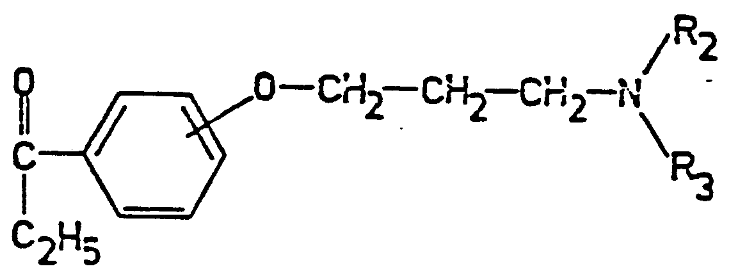

II ,

dans laquelle $R_2$ et $R_3$ sont tels que définis dans les revendications 1 ou 4 à 7, sur un composé organométallique comportant un groupe phényle substitué répondant à la formule générale

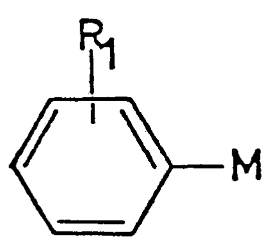

III ,

dans laquelle

$R_1$ est tel que défini dans les revendications 1 à 4,

M représente un atome de métal alcalin, de préférence un

atome de lithium, de potassium ou de sodium, ou un reste répondant à la formule MgX, avec

X = atome d'halogène, ou bien

b) on fait réagir des 1-{[3-(halo)-propoxy]-phényl}-1-{phényl}-propan-1-ols substitués répondant à la

formule générale

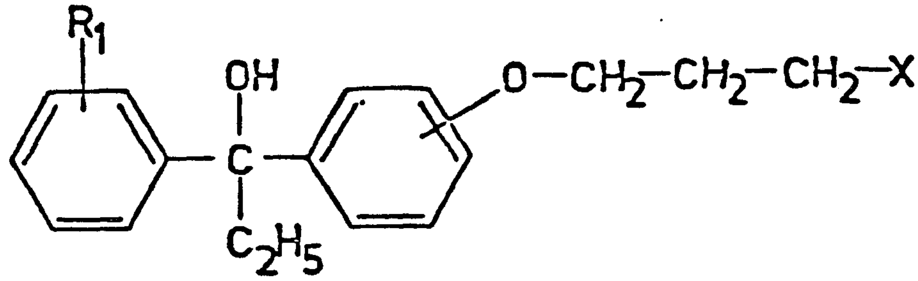

IV ,

dans laquelle
$R_1$ est tel que défini dans les revendications 1 à 4, et
X représente un atome d'halogène,
sur des amines secondaires répondant à la formule générale

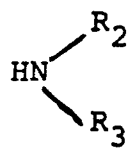

V ,

dans laquelle $R_2$ et $R_3$ sont tels que définis dans les revendications 1 ou 4 à 7, de préférence en présence d'agents de fixation d'acides, ou bien

c) on fait réagir des [3-(amino)-propoxy]-benzophénones répondant à la formule générale

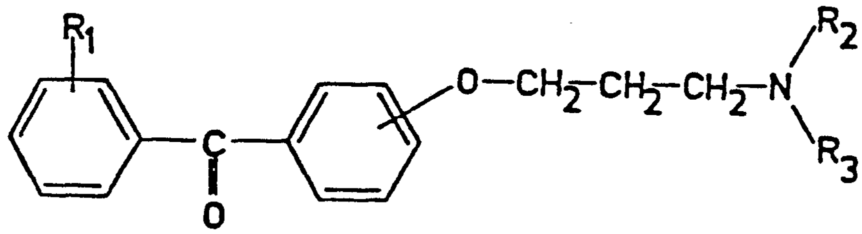

VI ,

dans laquelle $R_1$, $R_2$ et $R_3$ sont tels que définis dans les revendications 1 à 7, sur des composés organométalliques comportant un groupe éthyle, de préférence des halogénures d'éthylmagnésium ou de l'éthyllithium, ou bien

d) on fait réagir des propiophénones substituées sur le noyau benzénique répondant à la formule générale

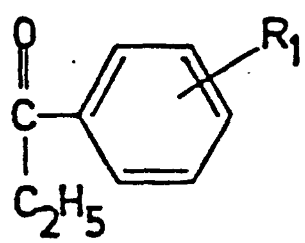

VII ,

dans laquelle $R_1$ est tel que défini dans les revendications 1 à 4, sur des composés de Grignard comportant un groupe {[3-(amino)-propoxy]-phényle} répondant à la formule générale

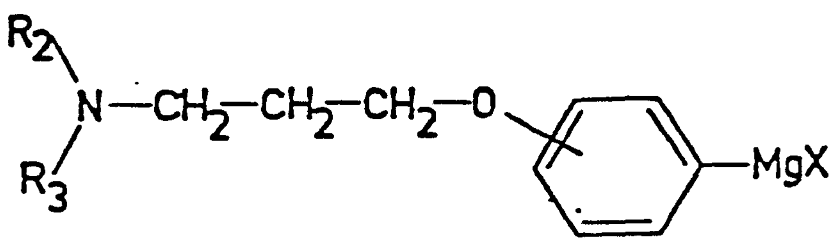

VIII. ,

dans laquelle
$R_2$ et $R_3$ sont tels que définis dans les revendications 1 ou 4 à 7, et
X représente un halogène, ou bien

e) on fait réagir des 1-(phényl)-1-(hydroxy-phényl)-propan-1-ols répondant à la formule générale

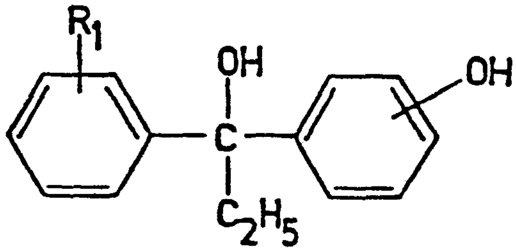

II ,

dans laquelle $R_1$ est tel que défini dans les revendications 1 à 4, de préférence sous la forme de leurs phénolates alcalins ou de leurs phénolates d'ammonium quaternaires, sur des amines tertiaires répondant à la formule générale

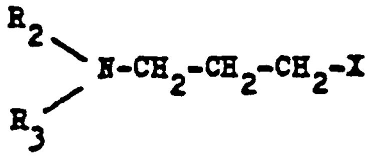

I ,

dans laquelle

$R_2$ et $R_3$ sont tels que définis dans les revendications 1 ou 4 à 7, et

X représente un reste alkylsulfonyloxy ou arylsulfonyloxy ou bien un atome d'halogène,

ou bien sur leurs sels, de préférence en présence d'agents de fixation d'acides,

puis l'on transforme éventuellement, de façon connue en soi, les 1-{[3-(amino)-propoxy]-phényl}-1-{phényl}-propan-1-ols répondant à la formule générale I, avec des acides en sels d'addition d'acides ou bien avec des agents de quaternisation en sels d'ammonium quaternaires, ou bien l'on transforme les sels d'addition d'acides des 1-{[3-(amino)-propoxy]-phényl}-1-{phényl}-propan-1-ols répondant à la formule générale I obtenus en les bases libres correspondantes répondant à la formule générale I et/ou en d'autres sels d'addition d'acides ou d'autres sels d'ammonium quaternaires.

10°) Médicaments, caractérisés en ce qu'ils renferment un ou plusieurs composé(s) selon les revendications 1 à 8, comme principe(s) actif(s), de façon appropriée en même temps qu'un ou plusieurs agent(s) de préparation maceutiques usuel.

**Revendications**

pour l'Etat contractant: AT

1. Procédé de préparation des 1-{[3(amino)-prop-oxy]-phényl}-1-{phényl}-propan-1-ols substitués répondant à la formule générale

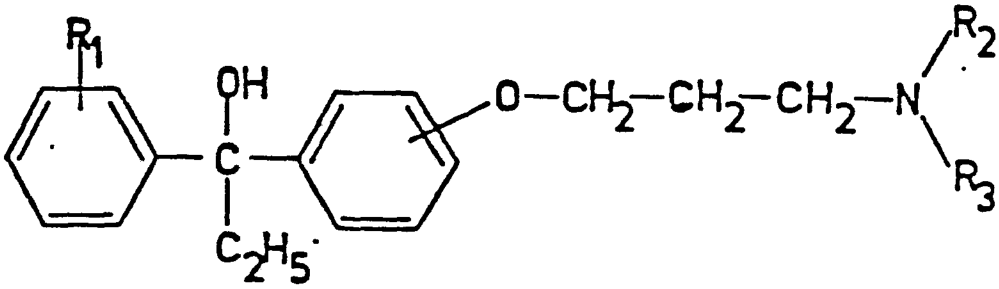

I ,

dans laquelle $R_1$ représente un atome d'halogène, un reste trihalométhyle ou un reste alcoxy ou alkyle comportant dans chaque cas 1 à 3 atome(s) de carbone,

$R_2$ représente un reste alkyle comportant 1 à 3 atome(s) de carbone, et

$R_3$ représente un reste cycloalkyle comportant 3 à 6 atomes de carbone, ou

$R_2$ et $R_3$ représentent, avec l'atome d'azote auquel ils sont liés, un reste hétérocyclique comportant au plus 8 maillons, éventuellement substitué par 1 ou plusieurs reste(s) alkyle comportant 1 à 4 atome(s) de carbone ou reste(s) benzylique(s) et/ou comportant éventuellement, comme autre hétéroatome, un atome d'oxygéne ou un autre atome d'azote,

ainsi que leurs sels d'addition avec des acides et sels quaternaires,

caractérisé en ce que

a) on fait réagir des propiophénones 3-(amino)-propoxysubstituées sur le noyau benzénique répondant à la formule générale

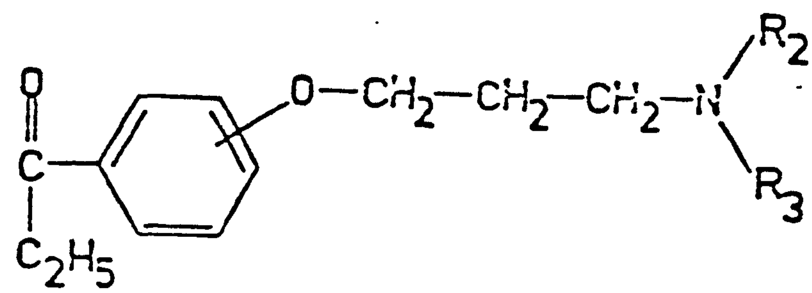

II ,

dans laquelle $R_2$ et $R_3$ sont tels que définis dans les revendications 1 ou 4 à 7, sur un composé organométallique comportant un groupe phényle substitué répondant à la formule générale

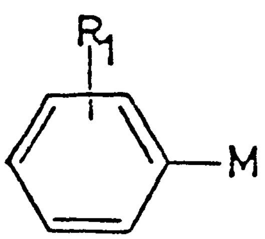

III ,

dans laquelle

$R_1$ est tel que défini dans les revendications 1 à 4,

M représente un atome de métal alcalin, de préférence un atome de lithium, de potassium ou de sodium, ou un reste répondant à la formule MgX, avec

X = atome d'halogène, ou bien

b) on fait réagir des 1-{[3-(halo)-propoxy]-phényl}-1-{phényl}-propan-1-ols substitués répondant à la formule générale

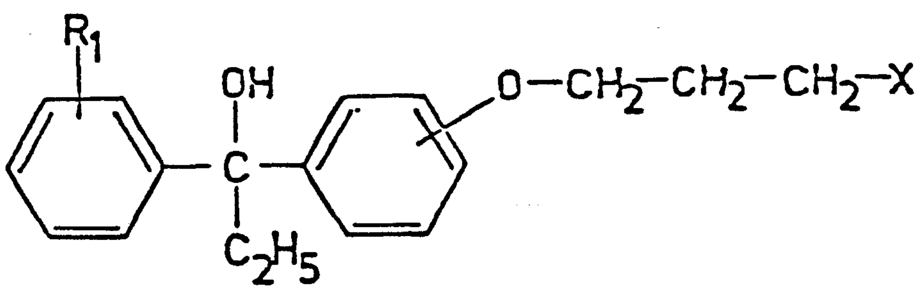

IV ,

dans laquelle

$R_1$ est tel que défini dans les revendications 1 à 4, et

X représente un atome d'halogène,

sur des amines secondaires répondant à la formule générale

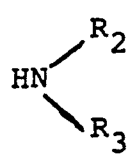

V ,

dans laquelle $R_2$ et $R_3$ sont tels que définis dans les revendications 1 ou 4 à 7, de préférence en présence d'agents de fixation d'acides, ou bien

c) on fait réagir des [3-(amino)-propoxy]-benzophénones répondant à la formule générale

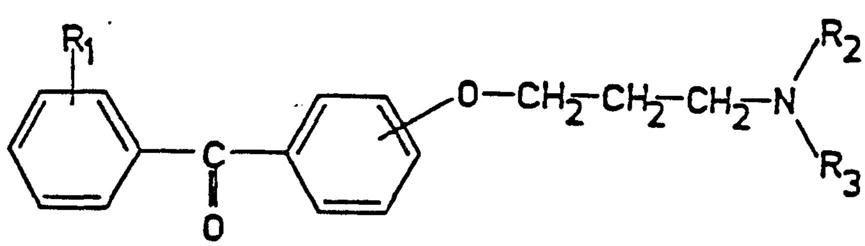

VI ,

dans laquelle $R_1$, $R_2$ et $R_3$ sont tels que définis dans les revendications 1 à 7, sur des composés organométalliques comportant un groupe éthyle, de préférence des halogénures d'éthylmagnésium ou de l'éthyllithium, ou bien

d) on fait réagir des propiophénones substituées sur le noyau benzénique répondant à la formule générale

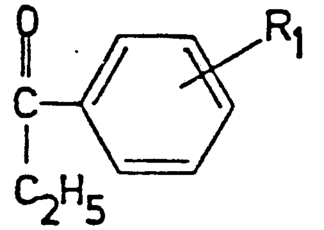

VII ,

dans laquelle $R_1$ est tel que défini dans les revendications 1 à 4, sur des composés de Grignard comportant un groupe {[3-(amino)-propoxy]-phényle} répondant à la formule générale

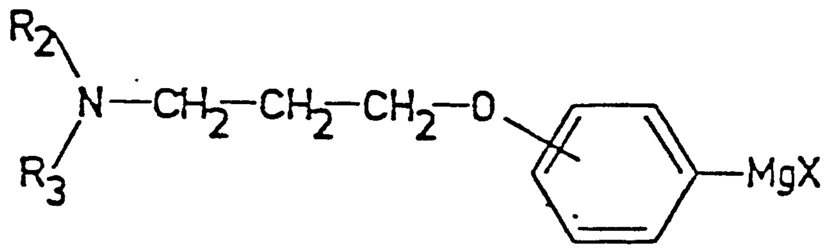

VIII ,

dans laquelle
$R_2$ et $R_3$ sont tels que définis dans les revendications 1 ou 4 à 7, et
X représente un halogène, ou bien
e) on fait réagir des 1-(phényl)-1-(hydroxy phényl)-propan-1-ols répondant à la formule générale

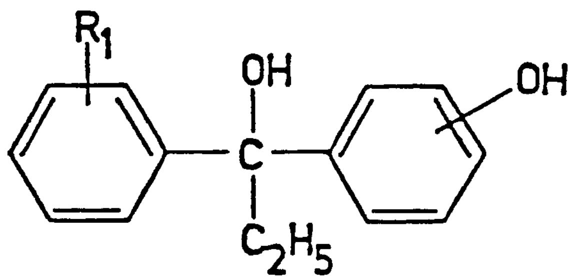

II ,

dans laquelle $R_1$ est tel que défini dans les revendications 1 à 4, de préférence sous la forme de leurs phénolates alcalins ou de leurs phénolates d'ammonium quaternaires, sur des amines tertiaires répondant à la formule générale

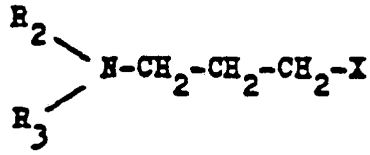

I ,

dans laquelle
$R_2$ et $R_3$ sont tels que définis dans les revendicationsl cu 4 à 7, et
X représente un reste alkylsulfonyloxy ou arylsulfonyloxy ou bien un atome d'halogène,
ou bien sur leurs sels, de préférence en présence d'agents de fixation d'acides,

puis l'on transforme éventuellement, de façon connue en soi, les 1-{[3-(amino)-propoxy]-phényl}-1-{phényl}-propan-1-ols répondant à la formule générale I, avec des acides en sels d'addition d'acides ou bien avec des agents de quaternisation en sels d'ammonium quaternaires, ou bien l'on transforme les sels d'addition acides des 1-{[3-(amino)-propoxy[-phényl}-1-{phényl}-propan-1-ols répondant à la formule genérale obtenus en les bases libres corresponsantes répondant à la formule générale I et/ou en d'autres sels d'addition d'acides ou d'autres sels d'ammonium quaternaires.

2. Procédé selon la revendication 1, charactérisés en ce que l'halogène que peut représenter $R_1$ est le chlore ou le fluor.

3. Procédé selon la revendication 1, charactérisés en ce que le reste trihalométhyle que peut représenter $R_1$

est un reste trifluorométhyle ou trichlorométhyle.

4. Procédé selon la revendication 1, charactérisés en ce que le reste alcoxy que peut représenter $R_1$ et/ou le ou les reste(s) atkyle que peut ou peuvent représenter $R_1$ et/ou $R_2$ est ou sont un ou des reste(s) comportant 1 ou 2, en particulier 1 atome(s) de carbone.

5. Procédé selon les revendications 1 à 4, charactérisés en ce que le reste cycloalkyle que peut représenter $R_3$ est un reste comportant 5 ou 6, en particulier 6, atomes de carbone.

6. Procédé selon les revendication 1 à 4, charactérisés en ce que le reste hétérocyclique que $R_2$ et $R_3$ peuvent représenter avec l'atome d'azote auquel ils sont liés est un reste comportant 3 à 8, en particulier 5 ou 6, tout particulièrement 6, atomes de carbone, surtout un reste morpholinyle, pipérazinyle ou imidazolyle.

7. Procédé selon les revendication 1 à 4 ou 6, charactérisés en ce que le ou les reste(s) alkyle par lequel ou lesquels le reste hétérocyclique que $R_2$ et $R_3$ peuvent représenter avec l'atome d'azote auquel ils sont liés peut être substitué, est ou sont un ou des reste(s) comportant 1 à 3,, en particulier 1 ou 2, tout particulièrement 1, atome(s) de carbone.

8. Procédé selon les revendication 1 à 7, charactérisés en ce que les 1-{[3-(amino)-propoxy]-phényl} -1-{phényl}-propan-1-ols suivants: le 1-(4-chlorophényl)-1-{4- [3-(4-méthyl-pipérazin-1-yl)-propoxy]-phényl}-propan-1-ol, le 1-(3-trifluorométhylphényl-1-{4-[3-(morpholin-1-yl)-propoxy]-phényl}-propan-1-ol, le 1-(4-fluorophé-nyl)-1-{4-[3-(4-benzyl-pipérazin-1-yl)-propoxy]-phényl}-propan-1-ol, le 1-(3-chlorophényl)-1-{4-[3-(4-benzyl-pipérazin-1-yl)-propoxy]-phényl}-propan-1-ol, le 1-(3-trifluorométhylphényl)-1-{4-[3-(4-méthyl-pipérazin-1-yl)-propoxy]-phényl}-propan-1-ol, le 1-(2-méthoxyphényl)-1-{4-[3 (4-méthyl-pipérazin-1-yl)-propoxy]-phényl}-propan-1-ol), le 1-(4-chlorophényl)-1-{4-[3-(4-benzyl-pipérazin-1-yl)-propoxy]-phényl}-propan-1-ol, le 1-(3-chlorophényl)-1-{4-[3-(4-méthyl-pipérazin-1-yl)-propoxy]-phényl}-pro-pan-1-ol, le 1-(2-méthoxyphényl)-1-{4-[3-(4-benzyl-pipé-razin-1-yl)-propoxy]-phényl}-propan-1-ol, le 1-(2-méthoxy-phényl)-1-{4-[3-(2-méthyl-imidazol-1-yl)-propoxy]-phényl} -propan-1-ol, le 1-(2-méthoxyphényl)-1- 4-[3-(imidazol-1-yl)-propoxy]-phényl}-propan-1-ol et le 1-{(3-trifluoro-méthylphényl)-1- 4-[3-(N-méthyl-N-cyclohexyl-amino)-propoxy]-phényl } -propan-1-ol, ainsi que leurs sels sont preparés.